(19) Europäisches Patentamt
European Patent Office
Office européen des brevets

(11) EP 1 280 090 A1

(12) **EUROPÄISCHE PATENTANMELDUNG**

(43) Veröffentlichungstag:
**29.01.2003 Patentblatt 2003/05**

(51) Int Cl.[7]: **G06F 19/00**, C12Q 1/68

(21) Anmeldenummer: **01118860.4**

(22) Anmeldetag: **16.08.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **27.07.2001 EP 01117707**

(71) Anmelder: **iSenseIt AG**
**28359 Bremen (DE)**

(72) Erfinder:
- **Nölte, Manfred**
  **27576 Bremerhaven (DE)**
- **Waschulzik, Thomas, Dr.**
  **28359 Bremen (DE)**
- **Volkmann, Gerald**
  **28203 Bremen (DE)**
- **Hofacker, Ivo, Dr.**
  **1170 Wien (AT)**
- **Rojek, Regina, Dr.**
  **28359 Bremen (DE)**
- **Blohm, Dietmar, Prof. Dr.**
  **27711 Osterholz-Scharmbeck (DE)**
- **Hildebrand, Martin**
  **28209 Bremen (DE)**
- **Peplies, Jörg**
  **28211 Bremen (DE)**

(74) Vertreter: **Goddar, Heinz J., Dr.**
**FORRESTER & BOEHMERT**
**Pettenkoferstrasse 20-22**
**80336 München (DE)**

(54) **Verfahren zur Konfiguration paralleler Nukleinsäureanalyseverfahren**

(57) Verfahren zur Konfigurierung paralleler Nukleinsäureanalyseverfahren zur Sequenzmengenklassifikation, Computerprogramm und paralles Nukleinsäureanalyseverfahren zur Sequenzmengen-Klassifikation.

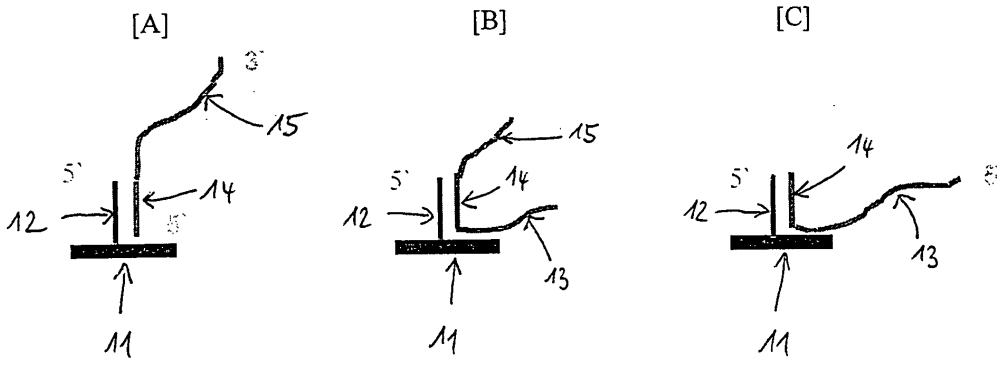


Figur 3



Printed by Jouve, 75001 PARIS (FR)

## Beschreibung

Die vorliegende Erfindung betrifft ein Verfahren zur Konfigurierung paralleler Nukleinsäureanalyseverfahren zur Sequenzmengenklassifikation, ein Computerprogramm zur Automatisierung dieses Konfigurierungsverfahrens und parallele Nukleinsäureanalyseverfahren zur Sequenzmengen-Klassifikation, die mittels demselben Konfigurierungsverfahren konfiguriert sind.

Die Konfiguration eines parallelen Nukleinsäureanalyseverfahrens beinhaltet eine Fänger-Nukleinsäuren-Bibliothek, Informationen über die Primer und das Protokoll eines gegebenenfalls vorgeschalteten Amplifikationsverfahrens und die Information, wie die Fänger-Nukleinsäuren an die Oberfläche anzubinden sind sowie zusätzliche Parameter für das Hybridisierungsprotokoll wie Ionen- und Formamid-Konzentrationen, Hybridisierungstemperatur, Hybridisierungszeit und -druck und die Parameter für das Protokoll der Nachbearbeitung, beispielsweise die Stringenz des Waschens der DNA-Microarrays. Für die Aufgabenstellungen paralleler Nukleinsäureanalyseverfahren ist es vorteilhaft Fänger-Nukleinsäuresequenzen-Bibilotheken zu konfigurieren und zu optimieren, um die technische Realisierung zu vereinfachen, zu beschleunigen, die Kosten für die Entwicklung zu reduzieren und um bei den Analysen möglichst gute Ergebnisse zu erzielen.

Eine nachzuweisende Nukleinsäure wird als Ziel-Nukleinsäure bezeichnet. Nukleinsäuren, die in der Probe auftreten können, aber keine Ziel-Nukleinsäuren sind, werden Nichtziel-Nukleinsäuren genannt. Die zu gewissen Bereichen der Ziel-Nukleinsäure teilweise komplementären, in dem Nukleinsäureanalyseverfahren zum Nachweis verwendeten Nukleinsäuresequenzen werden als Fänger-Nukleinsäuren bezeichnet. Eine Fänger-Nukleinsäure ist eine einzelsträngige oder erst im Laufe des Verfahrens einzelsträngig werdende Ribonukleinsäure oder Desoxyribonukleinsäure, die möglicherweise chemisch modifizierte Basen oder Basenanaloga, chemisch modifizierte Zucker oder Zukkeranaloga enthält oder anderweitig modifiziert ist und sich dadurch auszeichnet, dass sie mit hoher Spezifität und Selektivität an eine bestimmte, vorgegebene Nukleinsäure oder Klasse von Nukleinsäuren bindet. Unter Bibliothek soll im Folgenden eine Menge von Fänger-Nukleinsäuren verstanden werden.

Als parallele Nukleinsäureanalyseverfahren zur Sequenzmengen-Klassifikation werden in diesem Zusammenhang unter anderem der Einsatz von DNA-Microarrays, Microbeads und Molecular Beacons bezeichnet.

In der DNA-Analytik werden Fänger-Nukleinsäuresequenzen verwendet, um Organismen spezifisch zu identifizieren (K. Lindblad-Toh, E. Winchester, M. J. Daly, D. G. Wang, J. N. Hirschhorn, J.-P. Laviolette, K. Ardlie, D. E. Reich, E. Robinson, P. Sklar, N. Shah, D. Thomas, J.-B. Fan, T. Gingeras, J. Warrington, N. Patil, T. J. Hudson, and E. S. Lander: Largescale discovery and genotyping of single-nucleotide polymorphisms in the mouse, Nature Genetics, Vol. 24, April 2000). Werden diese Fänger-Nukleinsäuresequenzen in einer bestimmten Anordnung, beispielsweise matrixartig, dreiecks- oder sechsecksgitterartig ("orange pack") auf eine Oberfläche aufgebracht und durch eine vorbestimmte Oberflächenchemie gebunden, also immobilisiert, entstehen so genannte DNA-Mikroarrays (M. Schena (Editor). DNA Microarrays, Oxford University Press, 1999 **und** Niemeyer CM, Blohm D: DNA-Microarrays. Angew. Chem. Int. Ed. 1999, 38: 2865-2869. **und** Blohm DH and Guiseppi-Elie A: New developments in microarray technology. Current Opin. Biotechnol. 2001, **12**, 41-47). Werden Mengen dieser Fänger-Nukleinsäuresequenzen auf kleinen Partikeln immobilisiert, die entsprechend ihrer Beladung mit Fänger-Nukleinsäuresequenzen eindeutig identifizierbar sind, entstehen nukleinsäurebeladene Mikrobeads (S. Brenner, M. Johnson, J. Bridgham, G. Golda, D. H. Lloyd, D. Johnson, S. Luo, S. McCurdy, M. Foy, M. Ewan, R. Roth, D. George, S. Eletr, G. Albrecht, E. Vermaas, S. R. Williams, K. Moon, R. Burcham, M. Pallas, R. B. DuBridge, J. Kirchner, K. Fearon, J. Mao and K. Corcoran (2000): Gene expression analysis by massively parallel signature sequencing (MPPS) on microbead arrays. Nature Biotechnology, 18, 630 - 634.). Geben die Fänger-Nukleinsäuresequenzen erst nach einer Strukturveränderung ein Hybridisierungssignal, so handelt es sich um Molecular Beacons (Tyagi S and Kramer FR (1996) Molecular beacons: probes that fluoresce upon hybridization. Nat Biotechnol 14, 303-308.)

Diese parallelen Nukleinsäureanalyseverfahren zur Sequenzmengen-Klassifikation werden in einem Hybridisierungsprozess eingesetzt, wobei an ein Stück als Einzelstrang vorliegender Nukleinsäure des zu erkennenden Organismus ein kurzes passendes Gegenstück aus der Menge der Fänger-Nukleinsäuren gebunden wird. Diese Hybridisierung führt zu einem für die jeweilige Sequenzmenge spezifischen Hybridisierungssignal.

Unter DNA-Mikroarrays versteht man die räumlich definierte Anordnung von Fänger-Nukleinsäuren (z. B: DNA, RNA oder PNA) auf oder in einem festen oder Gel-artigen Träger unterschiedlichen Materials wie Glas-, Metall-, Halbleitermaterialien, Kunststoff. Die zu analysierende Probe enthält die Ziel-Nukleinsäure. Unter bestimmten experimentellen Bedingungen kommt es zwischen der Fänger-Nukleinsäure und der Ziel-Nukleinsäure zur Hybridisierung. Eine erfolgreiche Hybridisierung kann durch unterschiedliche Methoden detektiert werden, zum Beispiel mittels Fluoreszenz, Chemolumineszenz, Massenspektroskopie oder durch elektronische Sensorik, wobei die Messapparatur bereits ganz oder teilweise auf oder in dem Träger integriert sein kann. Die Fänger-Nukleinsäuren werden an definierten Positionen auf oder in dem Träger gebunden, wodurch das Hybridisierungssignal eindeutig einer Fänger-Nukleinsäure zugeordnet werden kann. Bei der Entwicklung von parallelen Nukleinsäureanalyseverfahren zur Sequenzmengen-Klassifikation ist die geeignete Festlegung der Sequenzen der Fänger-Nukleinsäuren von entscheidender Bedeutung

für die Leistungsfähigkeit des Verfahrens, wie geringe Störanfälligkeit, hohe Hybridisierungssensitivität und hohe Hybridisierungsspezifität.

Als Steigerung der Hybridisierungssensitivität soll bei sonst gleichen Versuchsbedingungen die Erhöhung des Anzahl von Fänger- und Ziel-Sequenzen, die miteinander eine Verbindung zum Doppelstrang eingehen, verstanden werden. Von einer Verbesserung der Hybridisierungsspezifität wird dann gesprochen, wenn unter sonst gleichen Versuchsbedingungen die Anzahl der Hybridisierungen von Fänger-Sequenzen mit Nichtziel-Sequenzen verringert wird.

Bisher wird die Auswahl der Fänger-Nukleinsäuresequenzen manuell oder mit einfachen Programmen durchgeführt; dieser Prozess ist sehr zeitaufwendig (Cynthia Gibas and Per Jambeck. Developing Bioinformatics Computer Skills, O'Reilley, April 2001) und die Fänger-Nukleinsäuresequenzen sind oft nur von sehr unzureichender Qualität (J. C. Williams, S. C. Case-Green, K. U. Mir and E. M. Southern (1994): Studies of oligonucleotide interactions by hybridisation to arrays: the influence of dangling ends on duplex yield. Nucleic Acids Research 22 (8): 1365 - 1367). Das bedeutet, dass bei Experimenten häufig die Nukleotidsequenzen nicht oder nicht mit der gewünschten Sensitivität nachgewiesen werden können, obwohl sich die gesuchte Sequenz in der Probe befindet. Ferner kann es durch Kreuzhybridisierungen zu falschpositiven Signalen kommen, nämlich dann, wenn die Fänger-Nukleinsäuren nicht nur mit der gesuchten Nukleinsäure, sondern auch mit anderen, ebenfalls in der Probe vorhandenen Nukleinsäuren eine Bindung eingehen.

Die bisher bekannten Verfahren zur Entwicklung von parallelen Nukleinsäureanalyseverfahren zur Sequenzmengen-Klassifikation weisen neben langen Entwicklungszeiten und hohen Entwicklungskosten auch erhöhte Entwicklungsrisiken sowie eine unzureichende Qualität der Ergebnisse bezüglich deren Anwendbarkeit und Leistungsfähigkeit in der Nukleinsäureanalytik auf.

Es ist daher Aufgabe der Erfindung, ein Verfahren bereitzustellen, mit dem mindestens ein vorgenannter Nachteil vermieden, zumindest aber reduziert wird.

Diese Aufgabe wird bei dem gattungsgemäßen Verfahren dadurch gelöst, dass (a) Fänger-Nukleinsäuren möglichst komplementär zu Bereichen von Ziel-Nukleinsäuren gewählt werden, die durch ihre Bindungsenergie oder Bindungswahrscheinlichkeit bei intramolekularen Wechselwirkungen oder Wechselwirkungen mit anderen beim Nachweis vorhandenen Molekülen ausgezeichnet sind

oder dass (b) Fänger-Nukleinsäuren möglichst komplementär zu Bereichen von Ziel-Nukleinsäuren gewählt werden, die in einem größeren Bereich der nachzuweisenden Nukleinsäuren enthalten sind, der ausgezeichnet ist durch seine Sekundärstruktur, quantifiziert durch die freien Energien der Sekundärstrukturen dieses Bereiches oder quantifiziert durch die Wahrscheinlichkeit der Sekundärstrukturbildung dieses Bereiches

oder dass (c) Fänger-Nukleinsäuren möglichst komplementär zu Bereichen von Ziel-Nukleinsäuren gewählt werden unter Berücksichtigung folgender Kriterien einzeln oder in Kombination:

> Datenbankqualität, Datenbanksensitivität, Datenbankspezifität, Positive Korrektheit bezüglich der Datenbank, Übereinstimmung, Rate der Falschnegativen bezüglich der Datenbank, Rate der Falschpositiven bezüglich der Datenbank, Negative Korrektheit bezüglich der Datenbank, Relative Falschheit bezüglich der Datenbank, Relativer Abstand der Positiven bezüglich der Datenbank, Datenbankdiversität, Redundanzniveau, Toleranzniveau, Distanzdifferenz, Hybridisierungsinitiierungsstelle, Anbindung der Fänger-Nukleinsäuren, relative Lage der Fänger-Nukleinsäuresequenzen zueinander, Zugänglichkeit der Ziel-Nukleinsäure durch die Fänger-Nukleinsäure, Ziel/Ziel-Dimere, Ziel/Nichtziel-Dimere, relative Lage der Fänger-Sequenzen auf der Ziel-Nukleinsäure und T-Gehalt;
>
> oder dass (d) Fänger-Nukleinsäuren möglichst komplementär zu Bereichen von Ziel-Nukleinsäuren gewählt werden und zweien oder allen dreien der oben unter (a) - (c) genannten Bedingungen genügen,
>
> wobei in allen der obigen vier Fälle (a) - (d) folgende Kriterien einzeln oder in Kombination zusätzlich Berücksichtigung finden können: Trefferanzahl, Bibliotheksgröße, Schmelztemperaturen, Sekundärstruktur der Fänger-Nukleinsäuren, Fänger/Fänger-Dimere, Spacer, GC-Klammerung.

In einer bevorzugten Ausführungsform wird die freie Energie $\Delta G$ der Bindungen der Ziel-Nukleinsäure und der Fänger-Nukleinsäuren, die bei intra- und intermolekularen Wechselwirkungen sowie Wechselwirkung mit anderen Molekülen entstehen, und die Wahrscheinlichkeit zur Sekundärstrukturbildung aus Messungen abgeleitet (Philip N. Borer, Barbara Dengler, Ignacio Tinoco: Stability of Ribonucleic acid Double-stranded Helices. *J. Mol. Biol.* **86** (1974), 843 - 853, **und** Matthew Petersheim, Douglas H. Turner: Base-Stacking and Base-Pairing Contributions to Helix Stability: Thermodynamics of Double-Helix Formation with CCGG, CCGGp, CCGGAp, ACCGGp, CCGGUp, and ACCGGUp. *Biochemistry* **22** (1983), 256 - 263, **und** John SantaLucia, Hatim T. Allawi, P. Ananda Seneviratne: Improved Nearest-Neighbor Parameters for Predicting DNA Duplex Stability. *Biochemistry* **35** (1996), 3355 - 3562, **und** John

SantaLucia: A unified view of polymer, dumbbell, and oligonucleotide DNA nearest-neighbor thermodynamics. *Proc. Natl. Acad. Sci. USA Biochemistry* **95** (1998), 1460 - 1465, deren jeweiliger Inhalt durch Bezugnahme hierin aufgenommen wird) oder durch ein computergestütztes Verfahren (M. Zuker, D. H. Mathews, D. H. Turner: Algorithms and Thermodynamics for RNA Secondary Structure Prediction: A Practical Guide. In: *RNA Biochemistry and Biotechnology* (Barciszewski & B.F.C. Clark, eds.), NATO ASI Series, Kluwer Academic Publishers (1999), pp. 11 - 43 **und** D. H. Mathews, J. Sabina, M. Zuker, D. H. Turner: Expanded Sequence Dependence of Thermodynamic Parameters Improves Prediction of RNA Secondary Structure. *J. Mol. Biol.* **288** (1999), 911 - 940, und I. L. Hofacker, W. Fontana, P.F. Stadler, S. Bonhoeffer, M. Tacker, P. Schuster: Fast Folding and Comparison of RNA Secondary Structures. Monatshefte f. Chemie 125 (1994), 167 - 188, und M. Zuker, P. Stiegler: Optimal computer folding of large RNA sequences using thermodynamic and auxiliary information. Nucl. Acid Res. **9** (1981), 133 - 148, **und** J. S. McCaskill: The equilibrium partition function and base pair binding probabilities for RNA secondary structures. *Biopolymers* **29** (1990), 1105 - 1119, deren jeweiliger Inhalt durch Bezugnahme hierin aufgenommen wird) bestimmt.

In einer weiteren Ausführungsform werden die Kriterien zumindest teilweise durch eine Auswahl von Teilsequenzen aus den komplementären Ziel-Nukleinsäuren erfüllt.

Bevorzugt werden die Kriterien zumindest teilweise durch gezielte Modifikationen von Teilsequenzen wie den Austausch von Basen, den Austausch von Zuckern, chemische Modifikation an diesen, Verwendung von Basenanaloga oder Zuckeranaloga, Veränderungen am Zukker-Phosphat-Rückgrat oder Modifikationen sonstiger Art erfüllt.

Besonders bevorzugt schließt die Konfigurierung die gezielte Amplifikation der Ziel-Nukleinsäuren zur Optimierung der in dieser Erfindung genannten Kriterien ein.

In einer weiteren Ausführungsform werden neben den Ziel-Nukleinsäuren und Fänger-Nukleinsäuren weitere Nukleinsäuren eingesetzt, die zusammen mit den Ziel-Nukleinsäuren und Fänger-Nukleinsäuren Multimere bilden, wodurch die Sekundärstruktur der Ziel-Nukleinsäuresequenz derart modifiziert wird, dass die Erfüllung der in Anspruch 1 genannten Kriterien begünstigt wird.

In einer weiteren Ausführungsform erfolgt die Anbindung der Fänger-Nukleinsäuren an den Träger derart, dass die Hybridisierungssensitivität optimiert wird.

In einer weiteren Ausführungsform wird die Datenbankqualität der Bibliothek über die Berechnung der Werte tp, tn, fp und fn bestimmt, die durch Einführung des Redundanz- und Toleranzniveaus modifizierbar sind.

Bevorzugt umfassen die computergestützte Verfahren zur Bestimmung der freien Energie $\Delta G$ und der Wahrscheinlichkeit für das Auftreten bestimmter Sekundärstrukturelemente die Berechnung der Zustandssumme aller möglichen Sekundärstrukturen oder Teilmengen hiervon oder die Berechnung der freien Energie der Sekundärstruktur mit der kleinsten freien Energie oder einer Teilmenge von Sekundärstrukturen mit einer freien Energie nahe der kleinsten freien Energie. Hierbei wird die Zustandssumme auch als *partition function* und die kleinste freie Energie auch als *mfe (minimal free energy)* bezeichnet.

In einer weiteren Ausführungsform werden die Spacer, mit denen die Fänger-Nukleinsäuren an den Träger angebunden werden, gezielt gewählt oder konstruiert.

Weiterhin wird die Aufgabe durch ein Computerprogramm mit Programmcodemitteln gelöst, um ein Verfahren gemäß der Erfindung durchzuführen, wenn dieses Programm auf einem Computer ausgeführt wird.

Insbesondere wird ein Computerprogramm mit Programmcodemitteln entsprechend Anspruch 11 bereitgestellt, die auf computerlesbaren Datenträgern gespeichert sind.

Schließlich wird die Aufgabe durch ein Computerprogramm-Produkt mit auf einem maschinenlesbaren Träger gespeicherten Programmcodemitteln gelöst, um alle Schritte gemäß einem der Ansprüche 1 bis 10 durchzuführen, wenn das Programm auf einem Computer ausgeführt wird.

Schließlich wird diese Aufgabe durch ein Nukeleinsäureanalyseverfahren zur Sequenzmengen-Konfigurierung gelöst, das nach einem Verfahren nach einem der Ansprüche 1 bis 10 konfiguriert ist.

Letztendlich kann diese Aufgabe durch eine Vorrichtung gelöst werden, die aufgrund ihrer konstruktiv fest vorgegebenen internen Struktur äußerlich das gleiche Verhalten wie ein Computerprogramm mit Programmcodemitteln nach Anspruch 11 zeigt. Beispielsweise kann diese Vorrichtung aus elektronischen Schaltkreisen bestehen (wie z. B. ASIC).

Der Erfindung liegt die überraschende Erkenntnis zugrunde, dass mit Hilfe der optimierten Konfigurierung, die durch Verwendung der in Anspruch 1 genannten Kriterien, die an Stelle einer direkten Optimierung nicht zugänglichen Störanfälligkeit, Hybridisierungssensitivität und Hybridisierungsspezifikation treten, parallele Nukleinsäureverfahren zur Sequenzmengen-Klassifikation mit einer verbesserten Leistungsfähigkeit erzielt werden können.

Figurenkurzbeschreibung

**Figur 1:** Es wird die Sekundärstruktur einer Ziel-Nukleinsäure dargestellt. Bei deren Amplifikation wird nur ein

Teilbereich vervielfältigt. Die Möglichkeiten zur Ausbildung von Sekundärstrukturen dieses Teilbereiches sind nach der Amplifikation eingeschränkt.

Figur 2: Es sind die Sekundärstruktur einer Ziel-Nukleinsäure sowie eine Fänger-Nukleinsäure dargestellt. Es wird nur der Bereich der Ziel-Nukleinsäure amplifiziert, mit dem eine Fänger-Nukleinsäure hybridisieren kann.

Figuren 3[A] bis [C]: Es sind an den Träger gebundene Fänger-Nukleinsäuren, die mit amplifizierten Ziel-Nukleinsäuren hybridiseren, dargestellt. In Figur 3[A] wurde der Primer so gewählt, dass die amplifzierte Ziel-Nukleinsäure mit ihrem Ende an die Fänger-Nukleinsäure derart bindet, dass der nicht von der Fänger-Nukleinsäure gebundene Teil der amplifizierte Ziel-Nukleinsäure vom Träger weg weist. In Figur 3[C] ist der nicht von der Fänger-Nukleinsäure gebundene Teil dem Träger zugewandt, während in Figur 3[B] die Ziel-Nukleinsäure mit ihrer Mitte an die Fänger-Nukleinsäure gebunden ist.

Figuren 4[A] und [B]: Eine Fänger-Nukleinsäure ist über ein Zwischenmolekül an den Träger gebunden. Die Hybridisierungsinitiierungsstelle befindet sich an dem Ende der Fänger-Nukleinsäure, das dem Träger zugewandt ist (Figur 4[A]). Die Hybridisierung mit der Ziel-Nukleinsäure wird dadurch eingeleitet, dass die sich Hybridisierungsinitiierungsstelle an einen Einzelstrangbereich der Zielnukleinsäure anlagert. Von hier ausgehend fügen sich Fänger-Nukleinsäure und Ziel-Nukleinsäure reißverschlussartig zusammen, wobei Doppelstrangbereiche aufgelöst werden (Figur [4]).

Nachfolgend werden mögliche Artgen der Berechnung und Verwendung der in Anspruch 1 genannten Kriterien beschrieben.

Datenbank-Qualität (DB-Qualität)

Die Datenbank-Qualität ist durch die vier Kenngrößen tp, fn, tn und fp bestimmt, die wie folgt definiert sind. Die Datenbank *N* sei die Menge aller Nukleinsäuren, die für eine gegebene Aufgabenstellung in Betracht gezogen werden müssen. *Z* sei die Menge aller Ziel-Nukleinsäuren. Damit ist dann *N - Z* die Menge aller Nichtziel-Nukleinsäuren. Die Menge *O* ist die Fänger-Nukleinsäuren-Bibliothek. Durch die Funktion *k* ist weiterhin jedem $o \in O$ eine frei wählbare Schranke *k*(*O*) zugeordnet. Hiermit wird nun für jedes $o \in O$ eine Menge von Nukleinsäuren Match (o,*k,N,A*) als Teilmenge von *N* definiert: $Match(o,k,N,A) := \{x \in N \mid \exists y{:}\, y = csubstr(x)$ und $A(y,o) \leq k(o)\}$, wobei csubstr(*x*) das Komplement einer zusammenhängenden Teilsequenz von *x* bezeichnet. Mit *A* wird eine Funktion bezeichnet, für die günstigerweise Hamming-Abstand, Levenstein-Abstand, Edit-Abstand oder Δ*G*-Abstand oder eine Kombination aus diesen gewählt wird.

Der Δ*G*-Abstand zwischen Fänger-Nukleinsäuresequenz o und Ziel-Nukleinsäuresequenz *x* ist definiert als:

$$\mathrm{dist}_{\Delta G}(o,x)=D(d_1(\Delta G(o,x),\Delta G(o',x)),d_2(\Delta G(o,x),\ \Delta G(o'',o)),d_3(\Delta G(o,x),\Delta G(o,x')))$$

wobei *o'* das Oligonukleotid ist, das exakt komplementär zu dem Bereich der Zielsequenz *x* ist, an den der Fänger bindet, o'' das Oligonukleotid ist, das exakt komplementär zu dem Oligonukleotid o ist und *x*' einer Sequenz entspricht, die im Bindungsbereich exakt komplementär zu o ist und ansonsten exakt der Zielsequenz *x* entspricht. $\Delta G(o,x)$, $\Delta G(o',x)$, $\Delta G(o'',x)$ und $\Delta G(o,x')$ sind die jeweiligen Hybridisierungsenergien. $d_1$, $d_2$ und $d_3$ sind beliebige Abstandsfunktion, insbesondere kann für diese

$d(\Delta G(o, y),\Delta G(o',y)) = \Delta G(o,y)-\Delta G(o',y)$ gewählt werden. *D* ist eine beliebige Funktion dreier Variablen, insbesondere kann für diese die gewichtete Summe der Variablen gewählt werden. Die Hybridisierungsenergie Δ*G* ist die freie Energie der Hybridisierungsreaktion und hängt nicht nur von den mit einander hybridisierenden Sequenzen, sondern auch von den Versuchsbedingungen wie Salzgehalt, Formamidgehalt, Temperatur und Druck ab. Die Berechnung der Hybridisierungsenergie kann mit den üblichen Verfahren durchgeführt werden (HYTER™ version 1.0, Nicolas Peyret and John SantaLucia, Jr., Wayne State University **und** SantaLucia (1998) *Proc. Natl. Acad. Sci. USA* 95, 1460 **und** Peyret et al. (1999) *Biochemistry 38,* 3468 - 3477, deren jeweiliger Inhalt durch Bezugnahme hierin aufgenommen wird).

Die vier Kenngrößen für die Datenbank-Qualität $DBQu(O,k_{tp},k_{fn},k_{tn},k_{fp},N)$ der Bibliothek *O* bezüglich der Datenbank *N* sind nun folgendermaßen definiert:

Die Anzahl der Richtig-Positiven: $tp(O,k_{tp},N,A) := |Z \cap \cup_{o \in O} Match(o,k_{tp}(o),N,A)|$,

die Anzahl der Falsch-Negativen: $fn(O, k_{fn}, N, A) := |Z - \cup_{o \in O} Match(o, k_{fn}(o), N,A)|$,

die Anzahl der Falsch-Positiven: $fp(O,k_{fp},N,A) := |(N - Z) \cap \cup_{o \in O} Match(o,k_{fp}(o),N,A)|$ und

die Anzahl der Richtig-Negativen: $tn(O,k_{tn},N,A) := |(N-Z) \cap \cup_{o \in O} Match(o,k_{tn}(o),N,A)|$.

Hierbei sind $k_{tp}$, $k_{fn}$, $k_{tn}$ und $k_{fp}$ Funktionen, mit denen in jedem der vier Fälle jeder Fänger-Nukleinsäuresequenz $o \in O$ unterschiedliche Schranken $k_{tp}(o)$, $k_{fn}(o)$, $k_{tn}(o)$ und $k_{fp}(o)$ zugeordnet sind. Bei der Berechnung von $tp(O, k_{tp}, N,A)$, $fn(O, k_{fn}, N,A)$, $fp(O,k_{fp},N,A)$ und $tn(O,k_{tn},N,A)$ kann während der algorithmischen Optimierung $k(o)$ jeweils individuell gewählt werden. So kann bei der Berechnung von $tp(O,k_{tp},N,A)$ eine exakte Sequenzübereinstimmung gefordert werden, während bei der Berechnung von $fp(O,k_{fp},N,A)$ ein oder mehrere Mismatches zulässig sind. Durch geeignete Wahl dieser Schranken für die algorithmische Optimierung kann letztendlich die tatsächliche Qualität des konfigurierten Nukleinsäureanalyseverfahrens verbessert werden. Hierbei ist jedoch zu beachten, dass $k(o)$ nicht so extrem gewählt werden darf, dass die anderen bei der Optimierung verwendeten Kriterien nicht mehr erfüllt werden können. Je größer $tp(O,k_{tp},N,A)$und $tn(O,k_{tn},N,A)$ und je kleiner fn $(O,k_{fn},N,A)$ und $fp(O,k_{fp},N,A)$ sind, desto besser ist die DB-Qualität der Bibliothek $O$.

Datenbanksensitivität (DB-Sensitivität)

Die DB-Sensitivität $DBSe(O,k_{tp},k_{fn},N,A)$ der Fänger-Nukleinsäuren-Bibliothek $O$ bezüglich der Datenbank N, einer Menge von Nukleinsäuren, gibt an, wie gut die Menge $Z$ der Ziel-Nukleinsäuren durch die Fänger der Bibliothek überdeckt wird.

$$DBSe(O,k_{tp},k_{fn},N,A) := \frac{tp(O,k_{tp},N,A)}{tp(O,k_{tp},N,A) + fn(O,k_{tp},N,A)}$$

Üblicherweise wird bei der algorithmischen Optimierung eine möglichst große DB-Sensitivität angestrebt. Es sind jedoch auch Fälle denkbar, bei denen eine möglichst kleine DB-Sensitivität angestrebt wird.

Datenbank-Spezifität DB-Spezifität)

Die DB-Spezifität $DBSp(O,k_{tn},k_{fp},N,A)$ der Fänger-Nukleinsäuren-Bibliothek $O$ bezüglich der Datenbank $N$, einer Menge von Nukleinsäuren, gibt an, wie groß der Anteil aller Nichtziel-Nukleinsäuren ist, an die keine Fänger-Nukleinsäuren binden.

$$DBSp(O,k_{tn},k_{fp},N,A) := \frac{tn(O,k_{tn},N,A)}{tn(O,k_{tn},N,A) + fp(O,k_{fp},N,A)}$$

Bei der algorithmischen Optimierung wird eine möglichst große DB-Spezifität angestrebt. Es sind jedoch auch Fälle denkbar, bei denen eine möglichst kleine DB-Spezifität angestrebt wird.

Positive Korrektheit bezüglich der Datenbank

Die Positive Korrektheit $DBpK(O,k_{tp},k_{fp},N,A)$ der Fänger-Nukleinsäuren-Bibliothek $O$ bezüglich der Datenbank N gibt an, wie groß der Anteil der von den Fängern der Bibliothek "erkannten" Nukleinsäuren ist, die tatsächlich in der die Menge Z der Ziel-Nukleinsäuren liegen.

$$DBpK(O,k_{tp},k_{fp},N,A) := \frac{tp(O,k_{tp},N,A)}{tp(O,k_{tp},N,A) + fp(O,k_{fp},N,A)}$$

Überlicherweise sollte diese Größe einen möglichst hohen Wert annehmen. Es sind jedoch auch Fälle denkbar, in denen sie einen möglichst kleinen Wert annehmen sollte.

Datenbank-Übereinstimmung

Die Datenbank-Übereinstimmung $DBUeb(O,k_{tp},k_{tn},N,A)$ der Fänger-Nukleinsäuren $O$ bezüglich der Datenbank N ist das Verhältnis von der Anzahl der von den Fängern der Bibliothek korrekt "erkannten" Ziel- und Nichtziel-Nukleinsäuren zu der Anzahl aller Sequenzen der Datenbank.

$$DBUeb(O,k_{tp},k_{tn},N,A) := \frac{tp(O,k_{tp},N,A) + tn\ (O,k_{tn},N,A)}{|N|}$$

Diese Größe sollte einen möglichst hohen Wert annehmen._Es sind jedoch auch Fälle denkbar, in denen sie einen möglichst kleinen Wert annehmen sollte.

Rate der Falschnegativen bezüglich der Datenbank

Die Rate der Falschnegativen DBRfn ($O,k_{fn},N,A$) der Fänger-Nukleinsäuren-Bibliothek *O* bezüglich der Datenbank *N* gibt an, wie groß der Anteil der von den Fängern der Bibliothek nicht "erkannten" Nukleinsäuren ist, die tatsächlich in der die Menge Z der Ziel-Nukleinsäuren liegen.

$$\mathrm{DBRfn}(O,k_{fn},N,A) := \frac{\mathrm{fn}(O,k_{fn},N,A)}{|Z|}$$

Diese Größe sollte einen möglichst kleinen Wert annehmen. Es sind jedoch auch Fälle denkbar, in denen sie einen möglichst kleinen Wert annehmen sollte.

Rate der Falschpositiven bezüglich der Datenbank

Die Rate der Falschpositiven DBRfp($O,k_{fp},N,A$) der Fänger-Nukleinsäuren-Bibliothek *O* bezüglich der Datenbank *N* gibt an, wie groß der Anteil der von den Fängern der Bibliothek "erkannten" Nukleinsäuren ist, die jedoch nicht in der die Menge *Z* der Ziel-Nukleinsäuren liegen.

$$DBRfp(O,k_{fp},N,A) := \frac{fp\ (O,\ k_{fp},\ N,\ A)}{|N\text{-}Z|}$$

Diese Größe sollte einen möglichst kleinen Wert annehmen._Es sind jedoch auch Fälle denkbar, in denen sie einen möglichst kleinen Wert annehmen sollte.

Negative Korrektheit bezüglich der Datenbank

Die Negative Korrektheit DBpK($O,k_{tn},k_{fn},N,A$) der Fänger-Nukleinsäuren-Bibliothek *O* bezüglich der Datenbank *N* gibt an, wie groß der Anteil der von den Fängern der Bibliothek nicht "erkannten" Nukleinsäuren ist, die tatsächlich nicht in der die Menge Z der Ziel-Nukleinsäuren liegen.

$$DBnK(O,k_{tn},k_{fn},N,A) := \frac{tn(O,k_{tn},N,A)}{tn(O,k_{tn},N,A) + fn(O,k_{fn},N,A)}$$

Diese Größe sollte einen möglichst hohen Wert annehmen. Es sind jedoch auch Fälle denkbar, in denen sie einen möglichst kleinen Wert annehmen sollte.

Relative Falschheit bezüglich der Datenbank

Die "relative Falschheit" DBrF(O,$k_{tp},k_{fp},N,A$) der Fänger-Nukleinsäuren-Bibliothek *O* bezüglich der Datenbank *N* gibt an, wie groß die Summe aus den von den Fängern nicht erfassten Ziel-Nukleinsäuren und den von den Fängern erfassten Nichtziel-Nukleinsäuren relativ zu allen Ziel-Nukleinsäuren ist:

$$DBrF(O,k_{fp},k_{fn},A) := \frac{fp(O,k_{fp},N,A) + fn(O,k_{fn},N,A)}{|Z|}$$

Der Wert dieses Kriterium sollte möglichst klein sein. Es sind jedoch auch Fälle denkbar, in denen er möglichst klein sein soll.

Relativer Abstand der Positiven bezüglich der Datenbank

Der relative Abstand der Positiven DBrAP($O$, $k_{tp}$, $k_{fp}$, $N$,$A$) bezüglich Fänger-Nukleinsäuren-Bibliothek $O$ bezüglich der Datenbank $N$ ist der Unterschied zwischen den von den Fängern erfassten Anteilen der Ziel-Nukleinsäuren und Nichtziel-Nukleinsäuren an ihren jeweiligen Mengen, also die Differenz von Datenbank-Sensitivität und Rate der Falschpositiven bezüglich der Datenbank

$$\mathrm{DBrAP}(O,k_{tp},k_{fp},N,A) := \frac{\mathrm{tp}(O,k_{tp},N,A)}{|Z|} - \frac{\mathrm{fp}(O,k_{fp},N,A)}{|N - Z|}$$

Dieses Kriterium ist, um gute Fänger-Nukleinsäuren-Bibliotheken, zu erhalten zu maximieren. In gewissen Fällen kann jedoch auch eine Minimierung sinnvoll sein.

Teilmengendiversität

Um die Abdeckung einer Menge $M$ *durch* Teilmengen $A_1$, $A_2$,... zu charakterisieren, wird die Teilmengendiversität eingeführt. Hierfür wird der Abstand $\mathrm{dist}_{set}$ zweier endlicher Mengen $A$ und $B$, eingeführt, der wenn $A$ gleich $B$ ist, gleich 0 ist und wenn $A$ und $B$ disjunkt sind, ein Maximum annimmt. Für $A=B=\emptyset$ wird generell $\mathrm{dist}_{set}(A,B)= \mathrm{dist}_{set}(\emptyset,\emptyset):= 0$ festgelegt. Bevorzugte Ausführungsbeispiele für derartige Abstände sind wenn $A \cup B \neq \emptyset$

$$\mathrm{dist}_{set}(A,B) = \frac{|A-B| + |B-A|}{|A \cup B|}$$

$$\mathrm{dist}_{set}(A,B) = \frac{\min\{|A-B|,|B-A|\}}{|A \cup B|}$$

$$\mathrm{dist}_{set}(A,B) = \frac{\max\{|A-B|,|B-A|\}}{|A \cup B|}$$

und entsprechende andere, wo der Nenner in obigen Definitionen durch 1, sonstige (positive) Konstanten oder, für den vorliegenden Fall weniger geeignet, durch $|A \cap B|$ ersetzt ist. Mit $\mathrm{dist}_{set}$ und bezüglich eines festgelegten $n$-tupels $M_A:=(A_1,A_2,...A_n)$ von Teilmengen von $M$ wird nun die Teilmengendiversität wie folgt definiert:

$$TMD(M_A) := \frac{\sum_{i=1}^{n} \sum_{j=1}^{n} \mathrm{dist}_{set}(A_i, A_j)}{n(n-1)}$$

Der Nenner kann hierbei auch durch einen anderen geeigneten Ausdruck, wie z. B.

$$\sum_{i=1}^{n} |A_i|$$

oder auch einfach 1 ersetzt werden.

Handelt es sich bei $M$ um eine Menge von Ziel-Nukleinsäuresequenzen und bei $O$ um eine zuvor festgelegte Menge von Fänger-Nukleinsäuren, so wird mit jedem Fänger $o \in O$ eine Teilmenge *match(o,k,M,A)* wie oben definiert festgelegt. Damit nimmt nun die Teilmengendiversität auf $M$ folgende Form an:

$$\mathrm{TMD}(O,k_{\mathrm{Di}},M,A):=\frac{\sum_{o\in O}\sum_{o'\in O}\mathrm{dist}_{\mathrm{set}}(\mathrm{match}(o,k_{\mathrm{Di}}(o),M,A),\mathrm{match}(o',k_{\mathrm{Di}}(o'),M,A))}{|O|(|O|-1)}$$

Hierbei ist $k_{\mathrm{Di}}$ die Funktion die jedem Fänger $o \in O$ eine individuelle Schranke $k_{Di}(o)$ zuordnet. Eine niedrige Teilmengendiversität bedeutet, dass die Oligonukleotide die Ziel-Nukleinsäuren in der Menge *M* nur wenig diskriminieren, während eine hohe Teilmengendiversität darauf hindeutet, dass die Fänger jeweils unterschiedliche Merkmale der Ziel-Nukleinsäuren "erkennen". Ersteres wäre z. B. der Fall, wenn die Fänger relativ spezifisch für Bereiche hoher Konserviertheit wären, während im zweiten Falle die Fänger eher an variable Bereiche der Ziel-Nukleinsäuren binden würden. Je nach Aufgabenstellung ist das Kriterium eine niedrige oder hohe Teilmengendiversität.

Datenbank-Diversität (DB-Diversität)

Mit obiger Teilmengendiversität kann die Datenbank-Diversität DBDi($O,k_{\mathrm{Di}},N,A$) der Bibliothek *O* bezüglich der Datenbank N, einer Menge von Nukleinsäuren, definiert werden. Diese gibt an, wie gut bei einer gegebenen Anzahl von Fänger-Nukleinsäuren die Menge der Nichtziel-Nukleinsäuren überdeckt wird.

Obige Datenbank N sei eine Hierarchie. Eine Hierarchie stellt ein Teilmengensystem derart dar, dass für je zwei Teilmengen $Z_1 \subseteq N$ und $Z_2 \subseteq N$ entweder gilt $Z_1 \cap Z_2 = \emptyset$ oder $Z_1 \subseteq Z_2$ oder Z2 $\subseteq Z_1$ gilt. Ein Beispiel für Hierarchien stellen Taxonomien dar, die bisweilen als Bäume dargestellt werden. Hierbei entspricht dann jedem Knoten des Baumes eine Menge. Die Kanten stehen für die Teilmengenbeziehung $\subseteq$, das heißt die dem Nachfolgerknoten entsprechende Menge ist Teilmenge der Menge, die dem Vorgängerknoten entspricht. Dies sei durch ein Beispiel verdeutlicht: Seien $Z_1,Z_2,Z_3 \subseteq Z_A$, wobei $Z_A$ in der Hierarchie über $Z_1$, $Z_2$ und $Z_3$ steht. In der entsprechenden Baumdarstellung sind $Z_1$, $Z_2$ und $Z_3$ dann (nicht unbedingt direkte) Nachfolgerknoten von $Z_A$. Die Hierarchie *N* lässt sich in eine Menge disjunkter Teilmengen zerlegen, derart, dass jede in der Hierarchie auftretende Menge die Vereinigung von solchen Teilmengen ist. Seien beispielsweise $Z_A$, $Z_1$, $Z_2$ und $Z_3$ Teilmengen von *N*, wobei $Z_1$, $Z_2$, $Z_3$ paarweise disjunkt und $Z_1,Z_2,Z_3 \subseteq Z_A$. Dann kann durch Einführen der Menge $Z_4 := Z_A - Z_1 - Z_2 - Z_3$ die Menge $Z_A$ als Vereinigung von $Z_1$, $Z_2$, $Z_3$ und $Z_4$ geschrieben werden. Taxonomische Hierarchien können bei der Konfigurierung nun dadurch berücksichtigt werden, dass für jede dieser (disjunkten) Teilmengen (im obigen Beispiel also $Z_1$, $Z_2$, $Z_3$ und $Z_4$) eine Oligobibliothek konfiguriert wird, wobei jedoch die Randbedingung einheitlicher Hybridisierungsparameter zu berücksichtigen sind. Im folgenden genügt es daher die weitere Betrachtung auf eine einzelne Menge Z zu beschränken.

Sei die Datenbank N die Menge aller Nukleinsäuren, die für eine gegebene Aufgabenstellung in Betracht gezogen werden müssen und sei Z die Menge aller Ziel-Nukleinsäuren. Ein in der Praxis häufig auftretendes Problem besteht darin, dass die Menge Z nicht vollständig bekannt ist, da die Menge *N* Nukleinsäuren enthält, deren Zugehörigkeit zu *Z* zum Beispiel aufgrund irrtümlicher oder unzureichender taxonomischer Klassifizierung nicht erkannt wurde. Es ist also tatsächlich nur eine Teilmenge $Z' \subseteq Z$ gegeben und somit kann auch nur eine Fänger-Nukleinsäuren-Bibliothek *O* für Z' und nicht, wie eigentlich erwünscht, für *Z* konfiguriert werden. Um in solchen Fällen eine Verbesserung bei der Optimierung zu erzielen, wird das Kriterium der Datenbank-Diversität (DBDi) einer Bibliothek eingeführt. Dieses Kriterium besagt, dass die in der Bibliothek enthaltenen Fänger-Nukleinsäuren einen möglichst großen Abstand zu einander haben sollen, wobei dieser Abstand über die durch die jeweilige Fänger-Nukleinsäure "abgedeckte" Teilmenge von *N - Z'* quantifiziert wird. Mit der oben eingeführten Teilmengendiversität wird nun die Datenbankdiversität wie folgt definiert:

$$\mathrm{DBDi}(O,k_{\mathrm{Di}},N,A):=\mathrm{TMD}(O,k_{\mathrm{Di}},N-Z',A)$$

$k_{\mathrm{Di}}$ ist eine Funktion, die für jede Fänger-Nukleinsäure $o \in O$, wie bereits auch an anderer Stelle, eine frei wählbare Schranke $k_{\mathrm{Di}}(o)$ liefert. In der Regel werden gute Fänger-Nukleinsäuren-Bibliotheken erhalten, wenn bei der Optimierung eine möglichst große Datenbankdiversität angestrebt wird. Es gibt aber Anwendungsfälle, bei denen eine möglichst geringe Datenbankdiversität anzustreben ist.

Distanzdifferenz

Mit der oben eingeführten Funktion *A* für ein Oligonukleotid o und einer Nukleinsäuresequenz *y* kann eine Funktion für ein Oligonukleotid o und einer Menge von Nukleinsäuren *Y* definiert werden: $A_{\mathrm{set}}(o,Y) := \Sigma_{y\in Y}\lambda(o,y)A(o,y)$ wobei $\lambda(o,y)$ ein für jede Kombination von Oligonukleotid *o* und Nukleinsäuresequenz *y* individuell wählbarer Gewichtungsfaktor ist. Hiermit wird nun die Distanzdifferenz DD($Y,o,Y'$) zwischen den Mengen *Y* und *Y'* für ein Oligonukleotid o wie folgt definiert:

$$DD(Y,o,Y') := \Sigma_{y \in Y} \lambda(o,y) A(o,y) - \sum_{y \in Y'} \lambda'(o,y) A(o,y)$$

In einer besonderen Ausführungsform wird für *Y* die Menge der Ziel-Nukleinsäuren, für *Y'* die Menge der Nichtziel-Nukleinsäuren und für A Hamming-Abstand, Levenstein-Abstand, Edit-Abstand, oder $\Delta G$-Abstand gewählt. Dabei wird eine möglichst große Distanzdifferenz zwischen der Menge der Ziel-Nukleinsäuren und der Menge der Nichtziel-Nukleinsäuren für die jeweils betrachteten Fänger-Nukleinsäuren angestrebt. In anderen Ausführungsformen kann es aber auch sinnvoll sein, eine geringe Distanzdifferenz anzustreben.

Redundanzniveau, Toleranzniveau

Das Redundanzniveau $r \geq 1$ gibt an, wie viele Fänger-Nukleinsäuren mit einer Ziel-Nukleinsäure hybridisieren sollen. Das Toleranzniveau *s*, $1 \leq s \leq r$, legt für eine Nicht-Zielsequenz fest, mit wie vielen Fänger-Nukleinsäuren sie hybridisieren darf, bevor sie entsprechend dem Toleranz-Kriterium berücksichtigt wird. Das Redundanzniveau in die oben angegebene vier Kenngrößen der Datenbankqualität zu integrieren geschieht dadurch, dass nicht mehr berücksichtigt wird, ob eine Ziel-Nukleinsäuren mit (mindestens) einer Fänger-Nukleinsäure hybridisert (sie also in $\bigcup_{o \in O}$ Match $(o,k(o),N,A)$ enthalten ist), sondern mit *wie vielen* Fänger-Nukleinsäuren sie jeweils hybridisieren kann. Entsprechend wird bezüglich des Toleranzniveaus mit den Nichtziel-Nukleinsäuren verfahren. Hierfür wird $t(x,O,k,N,A)$ eingeführt, die Anzahl der Fänger-Nukleinsäurene aus der Bibliothek *O*, die mit einer Nukleinsäure $x \in N$ hybridisieren. Formal

$$t(x,O,k,N,A) := \sum_{o \in O} |\{x\} \cap \mathrm{Match}(o,k(o),N,A)|$$

Hierbei ist *k* die Funktion, die jedem Fänger-Nukleinsäure $o \in O$ eine individuelle Schranke $k(o)$ zuordnet. Durch Einbeziehung des Redundanz- und Toleranzniveaus sind die vier Kenngrößen der Datenbank-Qualität wie folgt zu modifizieren:

Die Anzahl der Richtig-Positiven: $\mathrm{tp}(O,k_{tp},N,A,r,s) := |\{x \in Z | t(x,O,k,p,N,A) \geq r\}|$,
die Anzahl der Falsch-Negativen: $\mathrm{fn}(O,k_{fn},N, A,r, s) := |\{x \in Z \, | t(x,O,k_{fn},N,A) < r\}|$,
die Anzahl der Falsch-Positiven: $\mathrm{fp}(O,k_{fp},N,A,r,s) := |\{x \in N\text{-}Z | t(x,O,k_{fp},N,A) \geq s\}|$ und
die Anzahl der Richtig-Negativen: $\mathrm{tn}(O,k_{tn},N,A,r,s) := |\{x \in N - Z \, | t(x,O,k_{tn},N,A) < s\}|$.

Die obigen Definitionen von Datenbank-Sensitivität, Datenbank-Spezifität, positiver Korrektheit bezüglich der Datenbank, relativer Falschheit bezüglich der Datenbank und relativer Abstand der Positiven bezüglich der Datenbank sind entsprechend zu verallgemeinern. Zur Erzielung guter Ergebnisse werden bei der Optimierung Lösungen mit hohem Redundanzniveau und niedrigem Toleranzniveau angestrebt.

Trefferanzahl-Kriterium

Die Datenbank *N* sei die Menge aller Nukleotidsequenzen, die für eine gegebene Aufgabenstellung in Betracht gezogen werden müssen. Für jede Fänger-Nukleinsäure $o \in O$ und der ihr zugeordneten Schranke *k* und einer Abstandsfunktion *A* ist mit der oben definierten Menge Match$(o,k,N,A)$ auch die Anzahl tanz$(o,k,N,A)$ der Sequenzen $n \in N$ festgelegt, für die o = csubstr(*n*) ist, die also die zu o komplementäre Sequenz als fortlaufende Teilsequenz enthalten: tanz$(o,k,N,A)$ :=|Match$(o,k,N,A)$|. Diese Anzahl

$$\mathrm{dist}_{set}(A,B) = \mathrm{dist}_{set}(\emptyset,\emptyset) :=$$

stellt ein Maß für die Qualität des betreffenden Oligonukleotids dar. Die Trefferanzahl sollte überlicherweise möglichst groß sein. In gewissen Fällen kann auch eine kleine Trefferanzahl erwünscht sein.

Bibliotheksgröße

Die Bibliotheksgröße $|O|$ ist die Anzahl der Fänger-Nukleinsäuren, die in der Menge *O* enthalten sind. Bei der

Optimierung wird üblicherweise angestrebt, $|O|$ möglichst gering zu halten; unter Umständen kann auch eine maximale Bibliotheksgröße, die nicht überschritten werden darf, vorgegeben sein oder es kann auch das Ziel der Optimierung sein, $|O|$ zu maximieren.

Schmelztemperatur ($T_m$)

Die Berechnung der Schmelztemperatur $T_m$ ist Stand der Technik und kann mit verschiedenen Programmen durchgeführt werden (K. Breslauer, R. Frank, H. Blocker, L. Marky, "Predicting DNA duplex stability from the base sequence" Proc. Natl. Acad. Sci. USA 83 (1986), pages 3746 - 3750. **und** T. Xia, J. SantaLucia, M. E. Burkard, R. Kierzek, S. J. Schroeder, X. Jiao, C. Cox, D. H. Turner. Thermodynamic Parameters for an Expanded Nearest-Neighbor Modell for Formation of RNA Duplexes with Watson-Crick Base Pairs. Biochemistry, 37, p. 14719-14735, 1998, deren jeweiliger Inhalt durch Bezugnahme hierin aufgenommen wird). Kriterium ist eine möglichst einheitliche Schmelztemperatur entweder innerhalb von Teilmengen oder der Gesamtmenge der Hybride aus Fänger-Nukleinsäuren und ihren jeweiligen Ziel-Nukleinsäuren.

Exkurs: Größen zur Beschreibung von Sekundärstrukturen

Der Zustand *Y* eines thermodynamischen Systems ist eine Funktion der möglichen einzelnen Zustände.
$Y=F(s_1,s_2,\ldots s_n)$ wobei $s_k$ den *k*-ten von insgesamt n möglichen Zuständen des Systems bezeichnet.
Günstigerweise wird für *Y* die Zustandssumme *Z* gewählt, im wesentlichen ein Maß für die Energie einer thermodynamischen Gesamtheit, die im vorliegenden Falle die Menge aller möglichen Sekundärstrukturen umfasst: $Z = \Sigma_i \exp(\Delta G_i/kT)$, wobei die Summe über alle möglichen Zustände *i* zu bilden ist. Hierbei ist *k* die Boltzmann-Konstante und *T* die absolute Temperatur.
In einer weiteren bevorzugten Ausführungsform wird die minimale freie Energie (*mfe*) für die Bestimmung von *Y* verwendet.
Die *mfe*-Struktur ist diejenige Struktur die von allen möglichen Strukturen die kleinste freie Energie aufweist. Die Ermittlung dieser beiden Größen ist im Falle von DNA oder RNA Stand der Technik (M. Zuker, D.H. Mathews & D.H. Turner. Algorithms and Thermodynamics for RNA Secondary Structure Parameters: A Practical Guide. In RNA Biochemistry and Biotechnology, J. Barciszewski & B.F.C. Clark, eds., NATO ASI Series, Kluwer Academic Publishers, (1999). **und** D. H. Mathews, J. Sabina, M. Zuker & D.H. Turner. Expanded Sequence Dependence of Thermodynamic Parameters Provides Robust Prediction of RNA Secondary Structure. J. Mol. Biol. 228, 910 - 940 (1999). **sowie** I.L. Hofacker, W. Fontana, P.F. Stadler, S. Bonhoeffer, M. Tacker, P. Schuster (1994) Fast Folding and Comparison of RNA Secondary Structures. Monatshefte f. Chemie 125:167-188).
Sekundärstrukturen können ganz beträchtlich das Zustandekommen von Hybridisierungssignalen behindern. Sekundärstrukturen der Fänger-Nukleinsäure, wie auch der Ziel-Nukleinsäure zerstören die Zugänglichkeit, die für das Zustandekommen einer Hybridisierung zwischen diesen beiden Molekülen notwendig ist. Eine Sekundärstruktur mit einer großen Stabilität ist problematischer als eine mit einer geringen Stabilität, die in jeder natürlich vorkommenden Sequenz zu finden sein wird. Die Stabilität einer Sekundärstruktur wird durch $\Delta G$ erfasst. Je kleiner $\Delta G$ ist, umso stabiler ist die zugehörige Sekundärstruktur. Wenn $\Delta G$ negativ ist, ist eine Struktur um so stabiler, je größer der *Betrag* des entsprechenden $\Delta G$ ist.
Sei *S* die Menge aller Zustände $s_1, s_2, \ldots, s_{|S|}$, also die Gesamtheit der Zustände eines Systems. Sei weiterhin

$$E:\ 2^S \to \Re,\quad S' \mapsto E(S')$$

eine beliebige Funktion (nicht notwendigerweise total), die Teilmengen $S' \subseteq S$, also einer Gesamtheit von Zuständen, eine reelle Zahl $E(S')$ zuordnet, wobei $E(S')$ rechnerisch oder experimentell bestimmbar ist. Weiterhin genüge *E* einer Monotonie-Bedingung der Art, dass für alle Teilmengen $S', S'' \subseteq S$, für die *E* definiert ist, mit

$$S' \subseteq S'' \text{ gilt } E(S') \leq E(S'')$$

(Hierbei stellt die Verwendung der Relation $\leq$ keine Einschränkung dar, da gegebenenfalls *E* mit -1 multipliziert werden kann.)
Interessanterweise läßt sich nun aus einer für den einzelnen Zustand s definierten (qualitativen) Bedingung c und obiger Funktion *E* für eine Gesamtheit *S'* von Zuständen definierte (quantitative) Größe $C(S')$ gewinnen. Diese Größe stellt ein Maß dafür dar, inwieweit die Teilmenge *S'* als Gesamtheit der Bedingung c genügt. Der Vorteil dieser

Vorgehensweise liegt darin, dass bereits durch die Auswertung der Funktion $E$ für die Teilmenge $S' \subseteq S$ dieses Maß erhalten wird, ohne dass hierfür jeder Zustand $s \in S'$ einzeln betrachtet werden muss. Die Vorgehensweise ist im einzeln wie folgt. Sei $S'$ beliebige Teilmenge von $S$. Sei c eine für den einzelnen Zustand $s \in S'$ definierte Bedingung. Damit ist eine Menge $S'_c \subseteq S'$ definiert:

$$S'_c := \{s \in S | c(s)\}$$

(mit $c(.)$ als einstelligem Prädikat"... genügt der Bedingung $c$"). Durch den Vergleich von $E(S')$ mit $E(S'_c)$ ist nun ersichtlich, inwieweit $S'$ als Menge der Bedingung $c$ genügt. Als Vergleichsgröße $C(S')$ kann beispielsweise die Differenz:

$$C(S') := \Delta E_c(S') := E(S') - E(S'_c)$$

betrachtet werden. $\Delta E_c(S')$ ist um so kleiner, je mehr $S'$ als Gesamtheit der Bedingung $c$ genügt; insbesondere ergibt sich für $S' = S'_c$, dass $C(S') = \Delta E_c(S') = \Delta E_c(S'_c) = C(S'_c) = 0$ (Es sei jedoch angemerkt, dass nicht notwendigerweise aus $C(S') = \Delta E_c(S') = 0$ folgt, dass $S' = S'_c$.) Ein anderes Beispiel zur Realisierung des Vergleichs von $E(S')$ mit $E(S'_c)$ stellt die Quotientenbildung dar:

$$C(S') := E(S'_c)/E(S')$$

Dieser Quotient wird um so größer, je mehr $S'$ der Bedingung c genügt und wird gleich 1, wenn $S' = S'_c$. Die Realisierung dieser Vergleichsfunktion $C(S')$ kann jedoch den Bedürfnissen des konkreten Einzelfalles entsprechend erfolgen.

Insbesondere kann auch für $S'$ die gesamte Menge $S$ gewählt werden, wodurch sich eine quantifizierte Aussage $C(S)$ über die Gesamtheit in Bezug auf die Bedingung $c$ ergibt. Die Bedeutung dieses Verfahrens als Bestandteil der hier vorgelegten Erfindung liegt nun darin, dass nun verschiedene Bedingungen $c_1, c_2, \ldots$ in Bezug auf das System gegeneinander abgewogen werden können.

Im folgenden ist das jeweilige System ein Einzelstrang einer Nukleinsäure, wobei die Zustände dieses Systems die Sekundärstrukturen darstellen, die der Einzelstrang annehmen kann. Die Bedingungen $c$, die diesen Sekundärstruktur auferlegt werden, sind üblicherweise von der Art wie "der Bereich $k$ bis $l$ ist ungepaart", "der Bereich $k$ bis $l$ ist exakt komplementär zu einer vorgegeben Oligonukleotidsequenz", "der Bereich $k$ bis $l$ enthält einen zu einer vorgegebenen Oligonukleotidsequenz komplementären Teilbereich" usw. Zur Unterscheidung der entsprechenden Größen werden die Indizes "gesamt" und *"kl"* verwendet, also z. B. $S_{\text{gesamt}} := S$, $S_{kl} := S_c$ und mit obiger Funktion $E$ dann $E_{\text{gesamt}} := E(S_{gesamt}) := E(S)$ und $E_{kl} := E(S_{kl}) := E(S_c)$.

Nachfolgend soll auf drei Spezialfälle eingegangen werden:

a) $\Delta\Delta G$-Ansatz

Sei $S'$ eine Teilmenge der Menge $S_{\text{gesamt}}$ aller Sekundärstrukturen der Ziel-DNA. Für die Funktion $E$ wird $E(S') := -mfe(S') := -\min\{\Delta G(s) | s \in S'\}$ gewählt. Die Bedingung $c$ lautet, dass die Ziel-DNA an den Positionen $k$ bis $l$ ungepaart ist. Die Menge der Sekundärstrukturen, die dieser Bedingung genügen sei $S'_{kl}$. Für $C(S')$ wird

$$C(S') := \Delta E_{kl}(S') := E(S') - E(S'_{kl})$$

gewählt. Damit ist dann

$$\Delta\Delta G := C(S) := \Delta E_{kl}(S) := E(S) - E(S_{kl})$$

Diese Größe ist umso kleiner, je mehr der Bereich $k$ bis $l$ auf der Ziel-DNA als Einzelstrangbereich begünstigt ist. Es ist im übrigen auch möglich, an Stelle von $\Delta\Delta G$, das für die Menge $S$ aller Sekundärstrukturen berechnet wird, $\Delta\Delta G' = C(S')$ *zu* verwenden, wobei $S'$ eine geeignet gewählte Teilmenge von $S$ darstellt. "Geeignet gewählt" meint, dass $S'$ (im statistischen Sinne) repräsentativ für $S$ ist. Eine weitere Variante besteht darin, dass anstelle

der obigen Funktion für $E$ die mit $\lambda_i$ parametrisierte Funktion

$$E(S') := -\Sigma_{s_i \in S'} \lambda_i \Delta G(s_i)$$

verwendet wird. Übliches Ziel bei der Optimierung ist, $C(S')$ und $\Delta\Delta G$ möglichst gering zu halten. In speziellen Anwendungsfällen kann es aber auch das Ziel sein, daß $C(S')$ und $\Delta\Delta G$ möglichst groß sind.

b) Dotplot-Matrizen-Ansatz

Der Ansatz über Dotplot-Matrizen hat den Vorteil, dass die Berechnung der Funktion $E$ weniger aufwändig ist. Darüber hinaus können mehrere konkurrierende Sekundärstrukturen in diesem Kriterium bei der Berechnung von $E$ berücksichtigt werden. Sei $S'$ eine Teilmenge der Menge $S_{\text{gesamt}}$ aller Sekundärstrukturen der Ziel-DNA. Das Element $p_{ij}$, in der $i$-ten Zeile und $j$-ten Spalte mit $i < j$ dieser Dotplot-Matrix ist die Wahrscheinlichkeit dafür, dass Position $i$ mit Position $j$ eine Paarung eingeht, wenn alle Sekundärstrukturen der Menge $S'$ zugrundegelegt werden. Im folgenden sei $p_{ji} := p_{ij}$ für i > j und $p_{ii} := 0$. Die Bedingung c laute, dass die Ziel-DNA an den Positionen $k$ bis $l$ ungepaart ist. Die Menge aller Sekundärstrukturen, die dieser Bedingung genügen sei $S'_{kl} \subseteq S'$. Die Funktion $E$ auf diesen beiden Mengen ist als

$$E(S') = \sum_{i=1}^{n} \sum_{j=1}^{n} p_{ij}$$

bzw.

$$E(S'_{kl}) = \sum_{i=k}^{l} \sum_{j=1}^{n} p_{ij}$$

definiert, wobei $n$ die Sequenzlänge ist. Je weiter $E(S')$ und $E(S'_{kl})$ auseinander liegen, desto weniger ist der Bereich $k$ bis $l$ des Ziel-Moleküls bei der Bildung von Doppelsträngen involviert. Dies kann beispielsweise quantifiziert werden durch

a. $C(S') := E(S'_{kl})$

oder

b. $C(S') := E(S'_{kl}) - E(S_{gesamt})$

oder

c. $C(S') := E(S'_{kl}) / E(S')$

mit $E(S')>0$

Für $S'$ ist günstigerweise eine statistisch repräsentative Teilmenge von $S_{\text{gesamt}}$ oder die gesamte Menge *aller* Sekundärstrukturen $S_{\text{gesamt}}$ zu wählen. Dann tritt an Stelle der Menge $S'_{kl}$ die Menge $S_{kl}$. In diesem Fall können die Werte für $p_{ij}$ dann mit dem Verfahren von McCaskill (The equilibrium partition function and base pair binding probabilities for RNA secondary structure. *Biopolymers* **29** (1990), 1105 - 1119, deren Inhalt durch Bezugnahme hierin aufgenommen wird) bestimmt werden. Sie lassen sich in diesem Fall sogar als tatsächliche Wahrscheinlichkeiten für Basenpaarungen interpretieren. Kriterium ist, $C(S')$ möglichst gering zu halten. In speziellen Anwendungsfällen kann es aber auch das Ziel sein, daß $C(S')$ möglichst groß ist.

c) Ansatz über Zustandssummen

Dieses Verfahren kombiniert den energetischen $\Delta\Delta G$-Ansatz mit dem wahrscheinlichkeitsbasierten Dotplot-Matrizen-Ansatz. Sei $S'$ eine Teilmenge der Menge $S_{\text{gesamt}}$ aller Sekundärstrukturen der Ziel-DNA. Laute die Bedingung $c$, dass die Ziel-DNA an den Positionen $k$ bis $l$ ungepaart ist. Die Menge der Sekundärstrukturen, die

dieser Bedingung genügen sei $S'_{kl}$. Für die $S'$ bzw. $S'_{kl}$ können die jeweiligen Zustandssummen $Z(S')$ bzw. $Z(S'_c)$ oder die damit zusammenhängenden freien Energien $F(S')$ bzw. $F(S'_c)$ berechnet werden. Für $C(S')$ wird in der vorliegenden Ausführungsform $C(S'):=Z(S'_c)/Z(S')$ gewählt. In einer zweiten Ausführungsform werden die Zustandssummen über die freie Energie berechnet und $C(S') := Z(S'_c)/Z(S') = \exp(-F(S'_c)/kT)/\exp(-F(S')/kT)$, wobei $k$ die Boltzmann-Konstante und $T$ die absolute Temperatur ist. $C(S_{gesamt})$ kann dann als Wahrscheinlichkeit dafür angesehen werden, dass der Bereich $k$ bis $l$ im Zielmolekül als Einzelstrangbereich vorliegt. In einer alternativen Ausführungsform kann für $S'$ eine statistisch repräsentative Teilmenge von $S$ gewählt werden. Eine weitere Variante besteht darin, dass anstelle der Zustandsfunktion $Z(S')$ folgende parametrisierte Funktion

$$\widetilde{Z}(S') := \sum_{s_i \in S'} \lambda_i \exp(\Delta G(s_i)/kT)$$

verwendet wird. Kriterium ist, $C(S')$ möglichst gering zu halten. In speziellen Anwendungsfällen kann es aber auch das Ziel sein, daß $C(S')$ möglichst groß ist.

Sekundärstrukturen der Fänger-Nukleinsäuren

Durch ungünstige Sekundärstrukturen der Fänger-Nukleinsäuren kann deren Hybridisierung mit den Ziel-Nukleinsäuren erschwert werden. Die Berechnung der Wahrscheinlichkeit zur Ausbildung von Doppelstrangbereichen und der minimalen freien Energie aller Sekundärstrukturen des jeweiligen Fängers erfolgt mit den im vorigen Abschnitt "Größen zur Beschreibung von Sekundärstrukturen" genannten Verfahren. Bei der Optimierung wird üblicherweise angestrebt, dass die Fänger-Nukleinsäuren möglichst weitgehend als Einzelstrang vorliegen, dass also die Wahrscheinlichkeit zur Ausbildung von Doppelstrangbereichen möglichst gering ist und/oder die minimale freie Energie aller Sekundärstrukturen des jeweiligen Fängers möglichst groß ist. Es sind Anwendungsfälle denkbar, bei denen unter ausgezeichneten Hybridisierungsbedingungen die Fänger-Nukleinsäuren möglichst doppelsträngig vorliegen sollen.

Sekundärstruktur der Ziel-Nukleinsäuren

Durch ungünstige Sekundärstrukturen der Ziel-Nukleinsäuren kann deren Hybridisierung mit den Fänger-Nukleinsäuren erschwert werden. Die Berechnung der Wahrscheinlichkeit zur Ausbildung von Doppelstrangbereichen und der minimalen freien Energie aller Sekundärstrukturen des jeweiligen Fängers erfolgt mit den im obigen Abschnitt "Größen zur Beschreibung von Sekundärstrukturen" genannten Verfahren. Bei der Optimierung wird angestrebt, dass der Bereich der Ziel-Nukleinsäure, der weitgehend komplementär zu der Fänger-Nukleinsäure ist, möglichst weitgehend als Einzelstrang vorliegt, dass also die Wahrscheinlichkeit zur Ausbildung von Doppelsträngen, an denen dieser Bereich beteiligt ist, möglichst gering ist und/oder die minimale freie Energie aller Sekundärstrukturen dieses Bereiches möglichst groß ist. Es sind Anwendungsfälle denkbar, bei denen unter ausgezeichneten Hybridisierungsbedingungen die Ziel-Nukleinsäuren möglichst doppelsträngig vorliegen sollen.

Exkurs: Verfahren zur Bestimmung von Dimeren bzw. *n*-meren

Bezeichnen $A_1$, $A_2$, $A_3$ usw. unterschiedliche Moleküle. Zwei oder mehr Moleküle gleicher oder unterschiedlicher Art können sich aneinanderlagern. Die entstehenden Aggregate werden als Dimere oder Multimere bezeichnet. Im vorliegenden Fall sind diese Moleküle Nukleinsäuresequenzen, die mit einander hybridisieren. Dimere und Multimere beeinflussen die Bindung der Fänger-Nukleinsäuren an die Zielnukleinsäuren. Um ein Maß für die Wahrscheinlichkeit einer Dimer- (oder Multimer-)bildung zu erhalten, wird entsprechend den obigen Ansätzen, die auf der freien Energie, der Dotplot-Matrix bzw. der Zustandssumme basieren, verfahren. Allen drei Verfahren gemein ist, dass sowohl die einzelnen Sequenzen $A_1$, $A_2$, $A_3$ usw. jeweils für sich betrachtet werden, als auch Dimere der Form $A_1A_1$, $A_1A_2$, $A_1A_3$ usw., Trimere $A_1A_1A_1$, $A_1A_1A_2$, ..., $A_1A_2A_3$ usw. Da das jeweilige Multimer, da es aus mehreren einzelnen Strängen besteht, den obigen Verfahren nicht direkt zugänglich ist, wird es für die Berechnung durch einen entsprechenden Einzelstrang Y ersetzt. Dieser Einzelstrang enthält jeden der an der zu betrachteten Multimerbildung beteiligten Einzelstränge als Bestandteil, die jedoch noch durch hinreichend lange polyN-Sequenzen von einander getrennt sind. Hierbei steht N für spezielle Nukleotide, die keine Paarungen eingehen.

Als $A'_1$, $A'_2$, $A'_3$ usw. werden die Teilsequenzen des Strangs Y bezeichnet, die erhalten werden, wenn dieser innerhalb der polyN-Sequenzen "aufgespalten" wird. A' entspricht also der ursprünglichen Sequenz A, verlängert um eine gewisse Anzahl von Nucleotiden N, B' entspricht der ursprünglichen Sequenz B, der eine gewisse Anzahl von Nukleotiden N vorangeht und folgt, usw. Die Mengen ihrer Sekundärstrukturen seien $A'_1$, $A'_2$, $A'_3$ usw. Die Menge der berücksichtigten Sekundärstrukturen ist S', eine Teilmenge von $S_{gesamt}$, der Menge aller Zustände, also aller Sekundärstrukturen, die die Gesamtsequenz Y annehmen kann. Die Bedingung *c* lässt sich folgendermaßen formulieren: "es bestehen keine Bindungen zwischen Bereichen der Sequenz Y, die unterschiedlichen der Sequenzen $A_1$, $A_2$, $A_3$ usw. entsprechen". Wäre diese Bedingung für alle Sekundärstrukturen von Y erfüllt, würde dies bedeuten, dass $A_1$, $A_2$, $A_3$ usw. keine Di- bzw. Multimere bilden. Mit der Bedingung *c* sind die Teilmengen $S'_c \subseteq S'$ *und* $S_c \subseteq S_{\text{gesamt}}$ festgelegt. Mit der oben eingeführten Funktion *E* und der sich hieraus ergebenden Vergleichsfunktion *C* wird damit ein Maß dafür erhalten, wie stark $A_1$, $A_2$, $A_3$ usw. zur Multimerbildung neigen.

Nachfolgend soll auf drei Spezialfälle eingegangen werden:

a) $\Delta\Delta G$-Ansatz

Sei *S'* eine Teilmenge der Menge $S_{\text{gesamt}}$ aller Sekundärstrukturen der Ziel-DNA. Für die Funktion *E* wird $E(S'):=-mfe(S'):=-\min\{\Delta G(s)|s\in S'\}$ gewählt. Für $C(S')$ wird $C(S'):=\Delta E_c(S'):=E(S') - E(S'_c)$ gewählt. Damit ist dann $\Delta\Delta G:= C(S):=\Delta E_c(s):=E(S)-E(S_c)$ Hierbei kann die Berechnung von $E(S_c)$ mit den üblichen Verfahren durchgeführt werden, indem erst $E(A')$, $E(B')$, $E(C')$ usw. mit diesen berechnet werden und dann deren Summe gebildet wird, da $E(S_c)=E(A'_1)+E(A'_2)+E(A'_3)+...$ gilt. Diese Größe $\Delta\Delta G$ ist umso kleiner, je mehr die Wechselwirkung innerhalb der Bereiche $A_1$, $A_2$, $A_3$ usw. auf dem Strang Y gegenüber Wechselwirkungen zwischen diesen Bereichen begünstigt ist, also umsomehr Multimerbildung beungünstigt ist.

b) Dotplot-Matrizen-Ansatz

Das Element $p_{ij}$ in der *i*-ten Zeile und *j*-ten Spalte dieser Dotplot-Matrix ist die Wahrscheinlichkeit dafür, dass, bezogen auf die gesamte Menge S', Position *i* mit Position *j* der Sequenz Y eine Paarung eingeht. Die Funktion *E* auf den beiden Mengen $S'_c$ und *S'* ist als

$$E(S') = \sum_{i=1}^{n}\sum_{j=1}^{n} p_{ij} \text{ bzw.}$$

$$E(S'_c) = \sum_{i=k_1}^{l_1}\sum_{j=k_1}^{l_1} p_{ij} + \sum_{i=k_2}^{l_2}\sum_{j=k_2}^{l_2} p_{ij} + \sum_{i=k_3}^{l_3}\sum_{j=k_{C3}}^{l_3} p_{ij} + ...$$

definiert, wobei *c* die oben eingeführte Bedingung darstellt, *n* die Sequenzlänge ist und $k_1$, $k_2$, $k_3$,... Anfangs- und $l_1$, $l_2$, $l_3$,... Endpositionen der den Sequenzen $A_1$, $A_2$, $A_3$ usw. entsprechenden Bereichen auf dem Strang Y bezeichnen. Je weiter $E(S'_c)$ und $E(S'_c)$ liegen, desto weniger ist eine intermolekulare Hybridisierung zwischen den Strängen $A_1$, $A_2$, $A_3$, usw. zu erwarten. Dies kann beispielsweise quantifiziert werden durch

i. $C(S'):= E(S'_c)$

oder

ii. $C(S'):=E(S')-E(S')$

oder

iii. $C(S') := E(S'_c)/E(S')$

wobei zusätzlich $E(S') > 0$ zu fordern ist.

Günstigerweise wird für *S*' eine statistisch repräsentative Teilmenge von $S_{\text{gesamt}}$ gewählt. Noch besser ist es für *S'* die Menge *aller* Sekundärstrukturen $S_{\text{gesamt}}$ zu wählen. Dann tritt an Stelle der Menge $S'_c$ die Menge $S_c$. In diesem Fall können die Werte für $p_{ij}$ dann mit dem in dem Artikel "The equilibrium partition function and base pair binding probabilities for RNA secondary structure". *Biopolymers* **29** (1990), 1105 - 1119 beschriebenen Verfahren

von McCaskill, dessen Inhalt durch Bezugnahme hierin aufgenommen wird, bestimmt werden. Sie lassen sich in diesem Fall sogar als tatsächliche Wahrscheinlichkeiten für Basenpaarungen interpretieren.

c) Ansatz über Zustandssummen

Dieses Verfahren weist eine gewisse Analogie zum ΔΔG-Ansatz auf und bietet darüber die Vorteile des Dotplot-Matrizen-Ansatzes. Für die Mengen $S'$ bzw. $S'_c$ können die jeweiligen Zustandssummen $Z(S')$ bzw. $Z(S'_c)$ oder die damit zusammenhängenden freien Energien $F(S')$ bzw. $F(S'_c)$ berechnet werden. Für $C(S')$ wird in der vorliegenden Ausführungsform $C(S') := Z(S'_c) / Z(S')$ gewählt. In einer zweiten Ausführungsform werden die Zustandssummen über die freie Energie berechnet $C(S'):=Z(S'_c)/Z(S')=\exp(-F(S'_c)/kT)/\exp(-F(S')/kT)$. Günstigerweise wird für $S'$ die Menge $S_{gesamt}$ aller Sekundärstrukturen gewählt, so dass obige Zustandssummen mit den üblichen Verfahren berechnet werden können. $C(S')=C(S_{gesamt})$ kann dann als Wahrscheinlichkeit dafür angesehen werden, dass die Sequenzen $A_1$, $A_2$, $A_3$ usw. Multimere bilden.

In allen drei Spezialfällen wird üblicherweise angestrebt, Multimerbildung möglichst gering zu halten, also einen kleinen Wert für $C(S')$ zu erhalten. Es sind auch Fälle denkbar, in denen gezielte Multimerbildung angestrebt wird.

Ziel/Ziel-Dimere

Der Bereich, an den eine Fänger-Nukleinsäuresequenz an eine Ziel-Nukleinsäure bindet, sowie eine Umgebung dieses Bereiches sollen möglichst nicht an Hybridisierungen zwischen Ziel-Nukleinsäuren, also an Ziel/Ziel-Dimer-Bildung oder Multimer-Bildung zwischen Ziel-Nukleinsäuren beteiligt sein.

Ziel/Nichtziel-Dimere

Der Bereich, an den eine Fänger-Nukleinsäuresequenz an eine Ziel-Nukleinsäure bindet, sowie eine Umgebung dieses Bereiches sollen möglichst nicht an Hybridisierungen mit Nichtziel-Nukleinsäuren, also an Ziel/Nichtziel-Dimer-Bildung oder Multimer-Bildung zwischen Ziel-Nukleinsäuren und Nichtziel-Nukleinsäuren beteiligt sein.

Fänger/Fänger-Dimere

Fänger sollten möglichst keine Dimere oder Multimere bilden.

Relative Lage der Fänger-Sequenzen auf der Ziel-Nukleinsäure

Bei der Optimierung der relativen Lage der Komplemente der Fänger-Nukleinsäuren auf der Ziel-Nukleinsäure spielen folgende Kriterien eine Rolle:

i. die Verteilung der zu den Fänger-Nukleinsäuren komplementären Bereiche auf der Zielsequenz

ii. die Lage der zu den Fänger-Nukleinsäuren komplementären Bereichen auf der Ziel-Nukleinsäuresequenz in Bezug auf das 3'- oder 5'-Ende

iii. die Überschneidung der zu den Fänger-Nukleinsäuren komplementären Bereichen auf der Ziel-Nukleinsäuresequenz

iv. der Abstand der zu den Fänger-Nukleinsäuren komplementären Bereichen auf der Ziel-Nukleinsäuresequenz

Je nach Aufgabenstellung sind diese Kriterien einzeln oder in Kombination bei der Optimierung zu berücksichtigen, wobei jedes dieser Kriterien stark, schwach oder überhaupt nicht ausgeprägt sein kann. In einem besonderen Anwendungsfall dieser Kritierien, nämlich für die gezielte Amplifikation von Bereichen, wird gefordert, dass die zu den Oligonukleotiden komplementären Bereiche auf der Ziel-Nukleinsäure möglichst nahe beieinander liegen und sich überschneiden. In einem weiteren Anwendungsfall dieser Kriterien bei starker Fragmentierung der Ziel-Nukleinsäuren wird gefordert, dass die zu den Oligonukleotiden komplementären Bereiche auf der Ziel-Nukleinsäure möglichst weit auseinander liegen.

Kriterium der Spacer

Spacer sind die Moleküle mittels derer die Fänger-Nukleinsäuren an den Träger angebunden sind. Durch

geeignete Wahl der Spacer kann die Intensität des Hybridisierungssignals gezielt angepasst oder verbessert werden. Kriterium ist, dass die Fänger-Nukleinsäuren möglichst nicht mit den Spacern wechselwirken, insbesondere also, wenn es sich bei den Spacern um Nukleinsäuren handelt, dass die Fänger-Nukleinsäuren keine zu den Spacern komplementäre Teilbereiche enthalten.

Kriterium der GC-Klammerung

Es werden jene Fänger-Nukleinsäuren bevorzugt ausgewählt, die eine hohe Hybridisierungsenergie an den Enden der Fänger-Nukleinsäure bei deren Bindung an die Ziel-Nukleinsäure haben. Eine besondere Ausführungsform dieses Kriteriums lautet, dass die Fänger-Nukleinsäuren jeweils an beiden Enden einen hohen GC-Gehalt aufweisen.

Kriterium des hohen T-Gehalts

Es werden jene Fänger-Nukleinsäuren bevorzugt ausgewählt, die einen hohen T-Gehalt aufweisen. Dieses Kriterium ist insbesondere bei der Anbindung von Fänger-Nukleinsäuren über UV-Cross-linking von Bedeutung.

Sensitivitätsoptimierte Anbindung der Fängermoleküle

Die Anbindung der Fänger-Nukleinsäuren an den Träger erfolgt derart, dass die Hybridisierungssensitivität dadurch optimiert wird, dass die Ziel-Nukleinsäure während und nach der Hybridisierung möglichst vom Träger abgewandt ist. Eine Fänger-Nukleinsäure, deren komplementärer Bereich sich näher am 3'-Ende der Ziel-Nukleinsäure befindet, wird vorzugsweise mit dem 5'-Ende auf der Chip-Oberfläche angebunden, eine Fänger-Nukleinsäure, die näher am 5'-Ende der Ziel-Nukleinsäure bindet wird vorzugsweise mit dem 3'-Ende angebunden. (siehe auch Ausführungen zu den Figuren 3 [A - [C] unter der Überschrift "Gezielte Vervielfältigung der Ziel-Nukleinsäure (Polymerase-Kettenreaktion (PCR), strand displacement amplification (SDA) und andere Verfahren", Punkt 6.)

Hybridisierungsinitiierungsstelle

Es werden bevorzugt Fänger-Nukleinsäuren ausgewählt, deren Hybridisierungsinitiierungsstelle ein Bereich des Zielmoleküls mit schwach ausgeprägter Sekundärstruktur und hohem GC-Gehalt ist. Dieser Bereich zeichnet sich unter anderem durch die unter "Größen zur Beschreibung von Sekundärstrukturen" genannten Kriterien, wie möglichst große minimale freie Energie und möglichst geringe Wahrscheinlichkeit der Ausbildung von Sekundärstrukturelementen, aus. Die Fänger-Nukleinsäure wird so gewählt, dass das Komplement der Hybridisierungsinitiierungsstelle den Anfang dieser Fänger-Nukleinsäure bildet. Dabei wird als Anfang der Fänger-Nukleinsäure dessen Teil bezeichnet mit dem sie (unter Umständen über Zwischenmoleküle (Spacer)) an dem Träger befestigt ist. Zur weiteren Verbesserung der Sensitivität wird auch die räumliche Struktur der Ziel-Nukleinsäure berücksichtigt. Die Wahl der Hybridisierungsinitiierungsstelle erfolgt hierbei derart, dass insbesondere sie durch die Fänger-Nukleinsäure räumlich frei zugänglich ist, wie unten unter "Zugänglichkeit der Ziel-Nukleinsäure durch die Fänger-Nukleinsäure" ausgeführt.

Figuren 4 [A] und [B]: Eine-Hybridisierungsinitiierungsstelle 16 befindet sich an dem Ende der Fänger-Nukleinsäure 16 und 17, das einem Träger (nicht gezeigt) zugewandt ist. In der Figur ist der Fall dargestellt, dass die Anbindung der Fänger-Nukleinsäure an den Träger über ein Zwischenmolekül 18 erfolgt. Die Hybridisierung mit der Ziel-Nukleinsäure 19 - 20 wird dadurch eingeleitet, dass sich die Hybridisierungsinitierungsstelle 16 an einen Einzelstrangbereich 20 der Ziel-Nukleinsäure 19 - 20 anlagert. Von hier ausgehend fügen sich Fänger-Nukleinsäure 16 und 17 und Ziel-Nukleinsäure 19 -20 reißverschlussartig zusammen. Dieser Prozess kann sich bis in den Doppelstrangbereich 19 hinein fortsetzen, wobei die Bindungen zwischen ihren Einzelsträngen sukzessive aufgelöst werden.

Zugänglichkeit der Ziel-Nukleinsäure durch die Fänger-Nukleinsäure

Fänger-Nukleinsäuren werden als Komplemente von Bereichen der Ziel-Nukleinsäure gewählt, die im Raume (für eine Hybridisierung) frei zugänglich sind. Die Bestimmung der räumlichen Struktur kann entweder experimentell (Robert W. Holley, Jean Apgar, George A. Everett, James T. Madison, Mark Marquisee, Susan H. Merrill, John Robert Penswick, Ada Zamir: Structure of a Ribonucleic Acid. *Science* **147** (1965), 1462 - 1465. **und** J. D. Robertus, Jane E. Ladner, J. T. Finch, Daniela Rhodes, R. S. Brown, R. B. C. Clark, A. Klug: Structure of yeast phenylalanine tRNA at 3 Å resolution. *Nature* **250** (1974), 546 - 551. **und** H. W. Pley, K. M. Flaherty, D. B. McKay: Three-dimensional structure of a hammerhead ribozyme. *Nature* **372** (1994), 68 - 74. **und** W. G. Scott, J. T. Finch, A. Klug: Crystal structure of an all-RNA hammerhead ribozyme: a proposed mechanism for RNA catalytic cleavage. *Cell* **81** (1995), 991 - 1002 **und** J. H. Cate, A. R. Gooding, E. Podell, K. Zhou, B. L. Golden, C. E. Kundrot, T. R. Cech, J. A. Doudna: Crystal structure of a group I ribozyme domain: principles of RNA packing. *Science* **273** (1996), 1678 - 1685 **und** Ehresmann, F. Baudin,

M. Gougel, P. Romby, J. P. Ebel, B. Ehresmann. *Nucleic Acids Research* **15** (1987), 9109 **und** Knapp. In: *Methods in Enzymology — Volume 180 — RNA Processing — Part A — General Methods.* (James E. Dahlberg, John N. Abelson, eds.), Academic Press, Inc., San Diegeo, New York, Berkeley, Boston, London, Sydney, Tokyo, Toronto (1989), deren jeweiliger Inhalt durch Bezugnahme hierin aufgenommen wird) bestimmt werden oder über computergestützte Verfahren vorhergesagt werden (A. Leach: Molecular Modelling - Principles and Applications, Longman, 1996, **und** J. Setubal, J. Meidanis: Introduction to Computational Molecular Biology, PWS Publishing Company, 1997, **und** D. Frenkel, B. Smit: Understanding Molecular Simulation - From Algorithms to Applications, Academic Press, 1996 **und** J. A. McCammon, S. C. Harvey: Dynamics of proteins and nucleic acids, Cambridge University Press, 1987 **und** D. C. Rapaport: The Art of Molecular Dynamics Simulation. Cambridge, University Press 1997, deren Inhalt durch Bezugnahme hierin aufgenommen wird, **aber auch** z. B. http://www.iro.umontreal.ca/~major/mcsym.html, http://dasher.wustl.edu/tinker/).

Ausführungsbeispiel: Die Fänger-Nukleinsäure wird bei einer tRNA so gewählt, dass er im Anticodon-Arm der tRNA liegt, der aufgrund seiner räumlichen Struktur unter physiologischen Bedingungen besonders gut für Interaktionen mit einer Fänger-Nukleinsäure zugänglich ist.

Nachfolgend werden einige Unteransprüche näher erläutert.

Gezielte Multimer-Bildung

Neben den Fänger-Nukleinsäuresequenzen werden weitere Nukleinsäuresequenzen eingesetzt, die zusammen mit den Fänger- und Ziel-Nukleinsäuresequenzen Multimere bilden, wodurch die Sekundärstruktur der Ziel-Nukleinsäursequenz derart modifiziert wird, dass die Bindung der Fänger-Nukleinsäuresequenz an die Ziel-Nukleinsäuresequenz begünstigt wird. Durch geeignete Wahl der jeweiligen Schmelztemperaturen kann die Multimerbildung durch gezielte Änderung der Hybridisierungsbedingungen gesteuert werden.

Gezielte Vervielfältigung der Ziel-Nukleinsäure (Polymerase-Kettenreaktion (PCR), *strand displacement amplification* (SDA) und andere Verfahren).

Bei den hier zugrundegelegten Amplifikationsverfahren werden Nukleinsäursequenzen oder Teilbereiche von diesen vervielfältigt. Zur Unterscheidung dieser Nukleinsäuresequenzen von den bei dem Amplifikationsprozess entstehenden Produkten werden im folgenden erstere als *ursprüngliche* und letztere als *amplifizierte* Nukleinsäuresequenzen bezeichnet. Durch die Wahl der Primer können solche amplifizierten Ziel-Nukleinsäuren erhalten werden, die besonders günstige Eigenschaften bezüglich des zu konfigurierenden Nukleinsäurenachweisverfahrens aufweisen. Primer bestimmen den Anfang und das Ende des amplifizierten Bereiches. Die Auswahl der Primer erfolgt entsprechend den Kriterien für die Auswahl der Fänger-Nukleinsäuren unter der zusätzlichen Berücksichtigung folgender Kriterien:

1. Es werden genau die Bereiche der ursprünglichen Ziel-Nukleinsäuresequenz amplifiziert die Komplemente von besonders gut geeigneten Fänger-Nukleinsäuren enthalten.

2. Die amplifizierten Bereiche sollen möglichst eine vorgegebene Länge nicht überschreiten.

3. Die Anzahl der Primer soll möglichst gering sein.

4. Die Sekundärstrukturen der amplifizierten Ziel-Nukleinsäuren, sollten möglichst schwach ausgeprägt sein. Dies kann durch Erfüllung der oben unter "Größen zur Beschreibung von Sekundärstrukturen" genannten Kriterien, wie möglichst große minimale freie Energie und möglichst geringe Wahrscheinlichkeit der Ausbildung von Sekundärstrukturelementen, sicher gestellt werden.

Figur 1: Beispiel für die Sekundärstrukturausbildung der ursprünglichen Ziel-Nukleinsäure bestehend aus den Teilbereichen 1 bis 9: diese bildet zwei Schleifen: 2 - 4 und 6 - 8 mit den Doppelsträngen 2/4 und 6/8. Durch Wahl geeigneter Bedingungen bei der Amplifikation wird nur ein Teilbereich 3 - 7 der ursprünglichen Ziel-Nukleinsäure vervielfältigt. Wird dieser Teilbereich so gewählt, dass hierbei jeweils nur ein Strang (4 und 6) jedes Doppelstranges amplifiziert wird, so ist die Ausbildung obiger Doppelstränge 2/4 und 6/8 in der amplifizierten Ziel-Nukleinsäure 3 - 7 nicht mehr möglich. Dadurch wird dessen Sekundärstrukturbildung wesentlich eingeschränkt.

5. Die Sekundärstrukturen der amplifizierten Bereiche und insbesondere die Teilbereiche hiervon, die ein Komplement zu einer Fänger-Nukleinsäure enthalten, sollten möglichst schwach ausgeprägt sein. Dies kann durch Erfüllung der oben unter "Größen zur Beschreibung von Sekundärstrukturen" genannten Kriterien, wie möglichst große minimale freie Energie und möglichst geringe Wahrscheinlichkeit der Ausbildung von Sekundärstrukturelementen, sicher gestellt werden.

Figur 2: Eine Variante des Verfahrens unter 4. besteht darin, dass bei der Amplifikation keine globale Minimierung der Sekundärstruktur angestrebt wird, sondern diese auf den Bereich beschränkt wird, an den die Fänger-Nukleinsäure hybridisiert. Bindet der Fänger 10 beispielsweise im Bereich des rechten Doppelstranges 6/8 der ursprünglichen Ziel-Nukleinsäure 1 bis 9, so braucht die Möglichkeit zur Ausbildung der linken Schleife 2 - 4 in der amplifizierten Ziel-Nukleinsäure 3 - 7 nicht unterbunden zu werden.

6. Figuren 3 [A] - [C]: Die Primer werden so gewählt, dass die amplifzierten Ziel-Nukleinsäuren hinterher möglichst gut an die Fänger-Nukleinsäure binden können. Dies wird dadurch erreicht, dass der zu den Fänger-Nukleinsäure komplementäre Bereich der amplifizierten Ziel-Nukleinsäure möglichst nahe an dem Ende der Ziel-Nukleinsäure liegt, das nach der Hybridisierung dem Träger zugewandt ist. Die Figur 3[A] zeigt einen Träger 11, an den eine Fänger-Nukleinsäure 12 gebunden ist, derart daß die amplifizierte Ziel-Nukleinsäure 14 - 15 mit ihrem Ende 14 an die Fänger-Nukleinsäure 12 gebunden ist, so daß der restliche Teil 15 der amplifizierten Ziel-Nukleinsäure vom Träger 11 abgewandt ist. Die Zahlen "3'" und "5'" bezeichnen die Orientierung des Zucker-Phosphat-Rückgrats der jeweiligen Nukleinsäure. Bei den Figuren 3[B] und 3[C] wurde die amplifizierte Niel-Nukleinsäure 13 - 15 bzw. 13 - 14 so gewählt, daß diese noch einen zusätzlichen Bereich 13 aufweist, der bei der Bindung der amplifizierten Ziel-Nukleinsäure an die Fänger-Nukleinsäure 12 zum Träger 11 hingewandt ist. Dies führt zu sterischen Problemen, die bei in den Figuren 3 [B] und [C] dargestellten Fällen zu einer verringerten Hybridisierungssensitivität führen.

7. Die amplifizierten Bereiche werden so gewählt, dass sie möglichst wenig zu Ziel/Zieloder Ziel/Nichtziel-Dimer-Bildung neigen, wie oben unter "Verfahren zur Bestimmung von Dimeren bzw. *n*-meren" dargestellt.

<u>Gezielte Modifikation der Fänger-Nukleinsäuresequenzen durch Einbau von Mismatches</u>

Durch den gezielten Einbau von Mismatches in die oder chemischen Modifikationen der Fänger-Nukleinsäuren werden die in Anspruch 1 genannten Kriterien weiter optimiert.

1. Um eine Sekundärstrukturbildung der Fänger-Nukleinsäure weitgehendst zu vermeiden, wird durch den Austausch einzelner Basen in den doppelstrangbildenden Bereichen in der *mfe*-Sekundärstruktur diese gezielt destabilisiert. Zur Kompensation dieser Mismatches muss die Fänger-Nukleinsäure verlängert werden, damit ideale Hybridisierungsbedingungen im definierten Schmelztemperaturintervall erreicht werden. Zur Berechnung dieser Mismatch-Informationen und der Schmelztemperatur können beliebige Sekundärstruktur-Programme z. B. RNAheat oder mfold verwendet werden (J. S. McCaskill (1990) The equilibrium partition function and base pair binding probabilities for RNA secondary structures, Biopolymers 29: 1105 - 1119, **und** M. Zucker's mfold for DNA:www.bioinf.math.rpi.edu/~mfold/dna/forml.cgi **und** M. Zuker, P. Stiegler (1981) Optimal computer folding of large RNA sequences using thermodynamic and auxiliary information, Nucl Acid Res 9: 133 - 148, **und** SantaLucia, JJr (1998) A unified view of polymer, dumbbell, and oligonucleotide DNAnearestneighbor thermodynamics. Proc. Natl. Acad. Sci. USA 95, 1460-1465, deren Inhalt durch Bezugnahme hierin aufgenommen wird **und** Ivo Hofacker's ViennaRNA mit DNA-Paramtersatz: <u>www.tbi.univie.ac.at/~ivo/RNA/</u> **und** Hofacker, W. Fontana, P.F. Stadler, S. Bonhoeffer, M. Tacker, P. Schuster (1994); Fast Folding and Comparison of RNA Secondary Structures. Monatshefte f. Chemie 125: 167 - 188, wobei Inhalt von letzterem durch Bezugnahme hierin aufgenommen wird).

```
5'A A C G T A A A
3'A A G C A T A A
```

```
5'A A C G T A A A
3'A A G A A T A A
```

die viertletzte Base "A" wurde gezielt eingebaut, um die Fänger-Sekundarstruktur aufzubrechen (bzw. zu schwächen); ein geringeres $T_m$ kann durch Verlängerung des Fängers kompensiert werden.

2. Zur Optimierung der Sensitivität und zur Verringerung der Anzahl der benötigten Fänger-Nukleinsäuresequenzen kann eine mit entsprechendem Mismatch gezielt konstruierte Fänger-Nukleinsäuresequenz die Funktion von mehreren Fänger-Nukleinsäuresequenzen ausüben. Die verringerte Schmelztemperatur kann durch Verlängerung der Fänger-Nukleinsäure kompensiert werden.

...cgtAgtgttgGcgtacgcg...

...cgtAgtgttgGcgtacgcg...

...cgtAgtgttgGcgtacgcg...

...cgtTgtgttgAcgtacgcg...

...cgtTgtgttgAcgtacgcg... ...

...cgtTgtgttgAcgtacgcg... Target-Komplemente

---

...cgtAgtgtt... Fänger-Nukleinsäuresequenz1

---

...ttgAcgtacg... Fänger-Nukleinsäuresequenz2

...tAgtgttgAcgtacg... ← *ersetzt die oberen beiden Fänger-Nukleinsäuresequenzen*

---

3. Um eine Ziel-Nukleinsäure von einer Nichtziel-Nukleinsäure zu unterscheiden, die sich in nur wenigen Positionen von einander unterscheiden, ist es erforderlich, dass der von der Fänger-Nukleinsäure "abgedeckte" Bereich *alle* diese Positionen umfasst. Andernfalls würde nämlich die Fänger-Nukleinsäure auch mit der Nichtziel-Nukleinsäure relativ gut hybridisieren können und diese nicht mehr von der nachzuweisenden Ziel-Nukleinsäure unterschieden werden können. Liegen die Positionen, in denen sich Ziel- und Nichtziel-Nukleinsäure von einander unterscheiden, relativ weit auseinander, so sind die Fänger-Nukleinsäuren relativ lang, wodurch sich die Schmelztemperatur erhöht. Zur Anpassung dieser Schmelztemperatur $T_m$ wird diese durch den gezielten Einbau von Mismatches in die Fänger-Nukleinsäure verringert.

cgtTgtatcgatcgatcgttgTcgtacgcg ← Nichtziel-Nukleinsäure

unterscheidet sich von

cgtAgtatcgatcgatcgttgGcgtacgcg ← der Ziel-Nukleinsäure an

.........................................................den Positionen A und G

Da aufgrund des großen Abstandes zwischen A und G die Fänger-Nukleinsäuren zu lang und damit $T_m$ zu groß wäre, wird ein Mismatch (**c** an Stelle von **a**) eingebaut.

| | |
|---|---|
| Ziel-Nukleinsäur. | cgtAgtatcgatcgatcgttgGcgtacgcg |
| Teilbereich | cgtAgtatcgatcgatcgttgGcgt-3' |
| Fänger- Nukleinsäure: | gcaTcatagctagctagcaacCgca-5' |
| Fänger mit Mitsmatch: | gcaTcatagctcgctagcaacCgca-3' |

Einbau eines Mismatches „c" in den Fänger

4. Analog dem gezielten Einbau von Mismatches zur Verringerung der Bildung von Fänger-Nukleinsäure-Sekundärstrukturen kann die Stabilität von Dimeren und Multimeren gezielt vermindert werden.
5. Die Hybridisierungsenergie kann durch die gezielte Modifikationen von Teilsequenzen wie den Austausch von Basen, den Austausch von Zuckern, chemische Modifikation an diesen, Verwendung von Basenanaloga oder Zuckeranaloga, Veränderungen am Zucker-Phosphat-Rückgrat oder Modifikationen sonstiger Art nicht nur gezielt und feinjustierend verringert werden sondern auch gezielt vergrößert werden. Diese Verringerung der Bindungsstärke kann präziser als eingebaute Mismatches für die oben angegebenen technischen Anwendungen zur Verbesserung der Sensitivität und Spezifität sowie zur Reduktion der Stabilität von Fänger-Nukleinsäure-Sekundärstrukturen eingesetzt werden. Für hochvariable Virengenome ist es von Interesse mit kurzen Fänger-Nukleinsäuren zu arbeiten, da die stark konservierten Regionen oft nur sehr kurz sind. Deren Nachweis wird entweder mittels einer kurzen Fänger-Nukleinsäuren durchgeführt, oder durch eine größere Anzahl von Fänger-Nukleinsäuren, welche die Variabilität der nicht-konservierten Regionen mit abdecken müssen. Eine kurze Fänger-Nukleinsäure wird häufig die Schmelztemperatur der Bibliothek unterschreiten und muss daher durch gezielte chemische Modifikation an die Schmelztemperatur der Bibliothek angepasst werden.
VVVVVacggcttcGctacgctVVVVV ← "VVVV" ist jeweils eine hochvariable Region. "G" ist ein PNA-Guanin und vergrößert die Schmelztemperatur.

Wahl und Modifikation der Spacer

Spacer sind die Moleküle, mit denen die Fänger-Nukleinsäuren an den Träger gebunden werden. Diese werden gezielt gewählt, so dass die unten genannten Kriterien berücksichtigt werden. Handelt es sich bei den Spacern um Nukleinsäuren, so ist deren Sequenz entsprechend den folgenden Kriterien festzulegen, wobei der Spacer für jede Fänger-Nukleinsäure individuell gewählt werden kann.

1. Wechselwirkung mit den Fänger-Nukleinsäuren: Die Spacer sollen nicht an die Fänger-Nukleinsäuren binden. Handelt es sich beim Spacer um eine Nukleinsäure, so ist deren Sequenz so zu wählen, dass sie nicht komplementär zu der Sequenz der Fänger-Nukleinsäure ist. Die Neigung zur Dimer-Bildung zwischen Spacer und Fänger-Nukleinsäure kann gemäß obigen "Verfahren zur Bestimmung von Dimeren bzw. *n*-meren" ermittelt werden.

2. Wechselwirkungen mit Ziel-Nukleinsäuren: Die Spacer sollen nicht an die Ziel-Nukleinsäuren binden. Handelt es sich beim Spacer um eine Nukleinsäure, so ist deren Sequenz so zu wählen, dass diese zu allen Ziel-Nukleinsäuren nicht komplementär ist. Die Neigung zur Dimer-Bildung zwischen Spacer und Ziel-Nukleinsäure kann gemäß obigen "Verfahren zur Bestimmung von Dimeren bzw. *n*-meren" ermittelt werden.

3. Wechselwirkungen mit anderen vorhandenen Molekülen. Die Spacer sollen nicht mit anderen bei der Nachweisreaktion vorhandenen Molekülen eine Bindung eingehen oder reagieren. Insbesondere sollen sie nicht mit Nichtziel-Nukleinsäuren hybridisieren. Handelt es sich beim Spacer um eine Nukleinsäure, so ist deren Sequenz so zu wählen, dass sie nicht komplementär zu irgendeiner Nichtziel-Nukleinsäure ist. Die Neigung zur Dimer-Bildung zwischen Spacer und Nichtziel-Nukleinsäure kann gemäß obigen "Verfahren zur Bestimmung von Dimeren bzw. *n*-meren" ermittelt werden.

4. Beeinflussung der Schmelztemperatur. Die Spacer sollen nur einen möglichst kleinen Einfluss auf die Schmelztemperatur $T_m$ des Hybrids aus der über den Spacer an den Träger gebundenen Fänger-Nukleinsäure und der Ziel-Nukleinsäure haben.

5. Länge und Flexibilität des Spacers: Eine schlechte räumliche Zugänglichkeit der Ziel-Nukleinsäure durch die Fänger-Nukleinsäure kann durch einen besonders langen und flexiblen Spacer kompensiert werden. Weiterhin kann eine ungünstige Lage der Hybridisierungsinitiierungsstelle durch einen langen Spacer kompensiert werden. Als ungünstig wird die Lage der Hybridisierungsiniitierungsstelle hier angesehen, wenn diese sich in der Nähe des Trägers befindet oder die durch die Fänger-Nukleinsäure gebundene Ziel-Nukleinsäure sich zu nahe beim Träger befinden oder mit diesem kollidieren würde (siehe auch obigen Abschnitt "Hybridisierungsinitiierungsstelle"). Weiterhin kann auch eine ungünstige Lage des zu der Fänger-Nukleinsäure komplementären Bereiches auf der Ziel-Nukleinsäure durch einen längeren Fänger ausgeglichen werden. Diese ist gegeben, wenn nach der Hybridisierung mit der Fänger-Nukleinsäure die Ziel-Nukleinsäure dem Träger zugewandt ist oder sich hauptsächlich in der Nähe des Trägers befindet (siehe auch obigen Abschnitt "Sensitivitätsoptimierte Anbindung der Fängermoleküle). Im übrigen können durch einen hinreichend langen Spacer sämtliche Effekte neutralisiert oder zumindest abgemindert werden, die aufgrund einer Wechselwirkung zwischen Ziel-Nukleinsäure und Träger eine Bindung der Ziel-Nukleinsäure an die Fänger-Nukleinsäure erschweren oder gar unmöglich machen würden. Beispiele für

derartige Wechselwirkungen sind unter anderem elektrostatische Kräfte, elektrophoretische Effekte, oder auch nur einfache Kontakte zwischen Ziel-Nukleinsäure und Oberfläche aufgrund einer ungünstigen Geometrie letzterer. In den Fällen, wo die Wechselwirkung zwischen Ziel-Nukleinsäure und Träger eine Bindung der Ziel-Nukleinsäure an die Fänger-Nukleinsäure begünstigt, können kurze Spacer von Vorteil sein.

6. Sekundär- und Tertiärstruktur der Spacer: Die Spacer sollen eine Struktur aufweisen, die die Bindung der Ziel-Nukleinsäure an die Fänger-Nukleinsäure nicht behindert. Handelt es sich bei den Spacern um Nukleinsäuren, so ist deren Sequenz so zu wählen, dass diese möglichst als Einzelstrang vorliegt. Die Berechnung der Wahrscheinlichkeit zur Ausbildung von Doppelstrangbereichen erfolgt mit den im obigen Abschnitt "Größen zur Beschreibung von Sekundärstrukturen" genannten Verfahren.

7. Spacer/Spacer-Dimer-Bildung: Die Spacer sollten keine Dimere oder Multimere bilden. Handelt es sich bei den Spacern um Nukleinsäuren, so kann deren Neigung zur Dimer- oder Multimerbildung gemäß obigen "Verfahren zur Bestimmung von Dimeren bzw. *n*-meren" ermittelt werden

Durch das erfindungsgemäße Verfahren, zumindest jedoch in seiner besonderen Ausrührungsform wird bewirkt, dass die verbesserte Auswahl der bei einem parallelen Nukleinsäurenanalyseverfahren verwendeten Fänger-Nukleinsäuren dazu führt, dass

1. Arrays mit einer kleinen Menge an Fänger-Nukleinsäuren für eine vorgegebene Aufgabenstellung produziert werden können, da die Qualität der einzelnen Fänger-Nukleinsäuren besser ist (ein größerer Anteil der Fänger-Nukleinsäuren erfüllt seine Aufgabe wie geplant) und die Zusammenstellung der Fänger-Nukleinsäuren zu einer optimierten Bibliothek so durchgeführt wird, dass sich die Einzelkomponenten in ihrer Leistungsfähigkeit bezüglich der Aufgabenstellung geeignet ergänzen und Defizite einzelner Fänger-Nukleinsäuren durch andere Fänger-Nukleinsäuren ausgeglichen werden. Durch die geringere Anzahl der Fänger-Nukleinsäuren je Aufgabenstellung und parallelem Nukleinsäureanalyseverfahren werden die Kosten für die Erstellung sowohl von Prototypen als auch für das endgültige Produkt gesenkt.

2. die Entwicklungszeit und die Entwicklungskosten für ein Produkt zu einer vorgegebenen Aufgabenstellung reduziert werden können, da die langsame, fehlerträchtige und kostenintensive Tätigkeit der manuellen Konfigurierung einer Bibliothek durch einen schnellen, zuverlässigen und automatisierten Prozess ersetzt wird, der mehr Merkmale gleichzeitig berücksichtigen kann, als dies bei einem manuellen Prozess möglich wäre. Die Entwicklungskosten werden reduziert, da Arbeitszeit eingespart und Fehler vermieden werden. Die Anzahl der Entwicklungszyklen wird reduziert, weil die einzelnen Fänger-Nukleinsäuren besser sind, eine gezielt-kostenminimierende Redundanz bei der Konfigurierung eines Arrays einfach berücksichtigt werden kann und die Zusammenstellung der Bibliothek geeignet gewählt wird.

3. die Anwendbarkeit von parallelen Nukleinsäurenanalyseverfahren verbessert wird, da die Sensitivität des Arrays - aufgrund der nach besseren Kriterien ausgewählten Fänger-Nukleinsäuren - erhöht wird und durch die schnelle automatische Konfigurierung auch für komplexe Fragestellungen einfach, schnell, sicher und kostengünstig parallele Nukleinsäurenanalyseverfahren konfiguriert werden können.

4. Durch den Fortschritt bei der Konfigurierung und die damit verbundene Reduktion der Entwicklungszeiten und Kosten wird ebenfalls die Anwendbarkeit der Technologie entscheidend gesteigert.

Im folgenden wird eine besonders bevorzugte Ausführungsform des Verfahrens für die Konfigurierung paralleler Nukleinsäureanalyseverfahren beschrieben. Sämtliche Sequenzen einer bestimmten vorgegebenen Länge, die komplementär zu einem fortlaufenden Bereich einer Ziel-Nukleinsäure sind, stellen potentielle Fänger-Nukleinsäuren dar. Aus diesem werden diejenigen ausgewählt, die allen drei unter (a) - (c) von Anspruch 1 genannten Bedingungen sowie allen unter (c) und (d) von Anspruch 1 genannten Kriterien genügen. Dadurch, daß die Bedingungen und Kriterien nacheinander in der Reihenfolge des jeweils notwendigen Rechenaufwands angewendet werden, wobei mit den am wenigsten rechenaufwändigen Bedingungen und Kriterien begonnen wird, wird die Anzahl der potentiellen Fänger-Nukleinsäuren sukzessive verringert. Unter den so schließlich als Fänger-Nukleinsäure verbleibenden Kandidaten werden die tatsächlichen Fänger-Nukleinsäuren entsprechend den Ergebnissen einer Bewertung ausgewählt. Bei dieser Bewertung wird die Relevanz der einzelnen Bedingungen und Kriterien berücksichtigt. Die jeweilige Relevanz einer Bedingung und eines Kriteriums wird für jede Technologie-Plattform mittels eines Data-Mining-Verfahrens individuell ermittelt. Alternativ kommen insbesondere folgende Optimierungsalgorithmen zum Einsatz: Greedy-Strategien(Lawrence Hunter (Editor): Artificial Intelligence and Molecular Biology, MIT Press, 1993, deren Inhalt durch Bezugnahme

hierin aufgenommen wird), Genetischen Algorithmen (Neil Gershenfield; The Nature of Mathematical Modelling, Cambrigde University Press, 1999. **und** M. Mitchell: An Introduction to Genetic Algorithms, MIT Press, 1999 **und** David E. Goldberg: Genetic Algorithms in Search, Optimization, and Machine Learning. Reading, Massachusetts u. a. Orte, 1989, deren jeweiliger Inhalt durch Bezugnahme hierin aufgenommen wird), Evolutionären Strategien (Günter Bachelier: FitneßApproximationsmodelle in Evolutions-Strategien, Tectum Verlag, 1999, **und** Hartmut Pohlheim: Evolutionäre Algorithmen. Verfahren, Operatoren und Hinweise für die Praxis (VDI-Buch), Springer-Verlag Berlin Heidelberg, Oktober 1999, **und** Dipankar Dasgupta, Zbigniew Michalewicz: Evolutionary Algorithms in Engineering Applications, Springer Verlag, Mai 1997, deren jeweiliger Inhalt durch Bezugnahme hierin aufgenommen wird), Operations-Research-Algorithnmen (Frederick S. Hillier: Introduction to Operations Research, McGraw Hill College Div, August 2000, **und** Wolfgang Domschke, Andreas Drexl: Einführung in Operations Research (Springer-Lehrbuch), Springer Verlag, Mai 1998, **und** Werner Zimmermann: Operations Research. Quantitative Methoden zur Entscheidungsvorbereitung, Oldenbourg, München., 1999, deren Inhalt durch Bezugnahme hierin aufgenommen wird), Gradienten-Verfahren (Neil Gershenfield: The Nature of Mathematical Modelling, Cambrigde University Press, 1999. **und** M. Mitchell: An Introduction to Genetic Algorithms, MIT Press, 1999. **und** David E. Goldberg: Genetic Algorithms in Search, Optimization, and Machine Learning, Reading, Massachusetts u. a. Orte, 1989, deren Inhalt durch Bezugnahme hierin aufgenommen wird) und anderen numerische Verfahren zur Maximierung bzw. Minimierung von Bewertungsfunktionen (Philip E. Gill, Walter Murray, Margaret H. Wright: Practical Optimization. London u. a. Orte, 1981, **und** R. Fletcher: Practical Methods of Optimization. Second Edition. Chichester, u. a. Orte, 1987 und Byron S. Gottfried, Joel Weisman: Introduction to Optimizaton Theory. Englewood Cliffs, New Jersey, 1973, deren Inhalt durch Bezugnahme hierin aufgenommen wird). In den verwendeten Optimierungsverfahren werden die Kriterien in der entsprechend geeigneten Form angewendet.

Die in der vorangehenden Beschreibung sowie den Ansprüchen offenbarten Merkmale der Erfindung können sowohl einzeln, als auch in beliebiger Kombination für die Verwirklichung der Erfindung in verschiedenen Ausführungsformen wesentlich sein.

## Patentansprüche

1. Verfahren zur Konfigurierung paralleler Nukleinsäureanalyseverfahren zur Sequenzmengenklassifikation, **gekennzeichnet dadurch, dass** (a) Fänger-Nukleinsäuren möglichst komplementär zu Bereichen von Ziel-Nukleinsäuren gewählt werden, die durch ihre Bindungsenergie oder Bindungswahrscheinlichkeit bei intramolekularen Wechselwirkungen oder Wechselwirkungen mit anderen beim Nachweis vorhandenen Molekülen ausgezeichnet sind

   oder dass (b) Fänger-Nukleinsäuren möglichst komplementär zu Bereichen von Ziel-Nukleinsäuren gewählt werden, die in einem größeren Bereich der nachzuweisenden Nukleinsäuren enthalten sind, der ausgezeichnet ist durch seine Sekundärstruktur quantifiziert durch die freien Energien der Sekundärstrukturen dieses Bereiches oder quantifiziert durch die Wahrscheinlichkeit der Sekundärstrukturbildung dieses Bereiches

   oder dass (c) Fänger-Nukleinsäuren möglichst komplementär zu Bereichen von Ziel-Nukleinsäuren gewählt werden unter Berücksichtigung mindestens eines der folgenden Kriterien:

   Datenbankqualität, Datenbanksensitivität, Datenbankspezifität, Positive Korrektheit bezüglich der Datenbank, Übereinstimmung, Rate der Falschnegativen bezüglich der Datenbank, Rate der Falschpositiven bezüglich der Datenbank, Negative Korrektheit bezüglich der Datenbank, Relative Falschheit bezüglich der Datenbank, Relativer Abstand der Positiven bezüglich der Datenbank, Datenbankdiversität, Redundanzniveau, Toleranzniveau, Distanzdifferenz, Hybridisierungsinitiierungsstelle, Anbindung der Fänger-Nukleinsäuren, relative Lage der Fänger-Nukleinsäuresequenzen zueinander, Zugänglichkeit der Ziel-Nukleinsäure durch die Fänger-Nukleinsäure, Ziel/Ziel-Dimere, Ziel/Nichtziel-Dimere, relative Lage der Fänger-Sequenzen auf der Ziel-Nukleinsäure und T-Gehalt;

   oder dass (d) Fänger-Nukleinsäuren möglichst komplementär zu Bereichen von Ziel-Nukleinsäuren gewählt werden und zweien oder allen dreien der oben unter (a) - (c) genannten Bedingungen genügen,

   wobei in allen der obigen vier Fälle (a) - (d) folgende Kriterien einzeln oder in Kombination zusätzlich Berücksichtigung finden können: Trefferanzahl, Bibliotheksgröße, Schmelztemperaturen, Sekundärstruktur der Fänger-Nukleinsäuren, Fänger/Fänger-Dimere, Spacer, GC-Klammerung.

2. Verfahren nach Anspruch 1, **dadurch gekennzeichnet, dass** freie Energie und Wahrscheinlichkeit aus einer Messung abgeleitet werden oder durch computergestützte Verfahren bestimmt werden.

3. Verfahren nach Anspruch 1 oder 2, **dadurch gekennzeichnet, dass** die Kriterien zumindest teilweise durch eine Auswahl von Teilsequenzen aus den komplementären Ziel-Nukleinsäuren erfüllt werden.

4. Verfahren nach einem der vorangehenden Ansprüche, **dadurch gekennzeichnet, dass** die Kriterien zumindest teilweise durch gezielte Modifikationen von Teilsequenzen wie den Austausch von Basen, den Austausch von Zuckern, chemische Modifikation an diesen, Verwendung von Basenanaloga oder Zuckeranaloga, Veränderungen am Zucker-Phosphat-Rückgrat oder Modifikationen sonstiger Art erfüllt.

5. Verfahren nach einem der vorangehenden Ansprüche, **dadurch gekennzeichnet, dass** die Konfigurierung die gezielte Amplifikation der Ziel-Nukleinsäuren zur Optimierung der in den Ansprüchen genannten Kriterien einschließt.

6. Verfahren nach einem der vorangehenden Ansprüche, **dadurch gekennzeichnet, dass** neben den Ziel-Nukleinsäuren und Fänger-Nukleinsäuren weitere Nukleinsäuren eingesetzt werden, die zusammen mit den Ziel-Nukleinsäuren und Fänger-Nukleinsäuren Multimere bilden können, wodurch die Sekundärstruktur der Ziel-Nukleinsäuresequenz derart modifiziert wird, dass die Bindung der Fänger-Nukleinsäuresequenz an die Ziel-Nukleinsäuresequenz begünstigt wird.

7. Verfahren nach einem der vorangehenden Ansprüche, **dadurch gekennzeichnet, dass** die Anbindung der Fänger-Nukleinsäuren an den Träger so erfolgt, dass die Hybridisierungssensitivität optimiert wird.

8. Verfahren nach einem der vorangehenden Ansprüche, **dadurch gekennzeichnet, dass** die Datenbankqualität der Bibliothek über die Berechnung der durch die Einführung des Redundanz- und Toleranzniveaus modifizierbaren Werte tp, tn, fp und fn bestimmt wird.

9. Verfahren nach einem der vorangehenden Ansprüche, **dadurch gekennzeichnet, dass** die computergestützten Verfahren zur Bestimmung der freien Energie und der Wahrscheinlichkeit von Sekundärstrukturelementen die Berechnung der Zustandssumme aller möglichen Sekundärstrukturen oder Teilmengen hiervon oder die Berechnung der freien Energie der Sekundärstruktur mit der kleinsten freien Energie oder einer Teilmenge von Sekundärstrukturen mit einer freien Energie nahe der kleinsten freien Energie umfasst.

10. Verfahren nach einem der vorangehenden Ansprüche, **dadurch gekennzeichnet, dass** die Spacer, mit denen die Fänger-Nukleinsäuren an den Träger angebunden werden, gezielt gewählt oder konstruiert werden.

11. Computerprogramm mit Programmcodemitteln, um ein Verfahren gemäß einem der vorangehenden Ansprüche durchzuführen, wenn das Programm auf einem Computer ausgeführt wird.

12. Computerprogramm mit Programmcodemitteln nach Anspruch 11, die auf einem computerlesbaren Datenträger gespeichert sind.

13. Computerprogramm-Produkt mit auf einem maschinenlesbaren Träger gespeicherten Programmcodemitteln, um alle Schritte gemäß einem der Ansprüche 1 bis 10 durchzuführen, wenn das Programm auf einem Computer ausgeführt wird.

14. Vorrichtung, die aufgrund ihrer konstruktiv fest vorgegebenen internen Struktur das gleiche Verhalten wie ein Computerprogramm mit Programmcodemitteln nach Anspruch 11 zeigt.

15. Paralleles Nukleinsäureanalyseverfahren zur Sequenzmengen-Klassifikation, das nach einem Verfahren nach einem der Ansprüche 1 bis 10 konfiguriert ist.

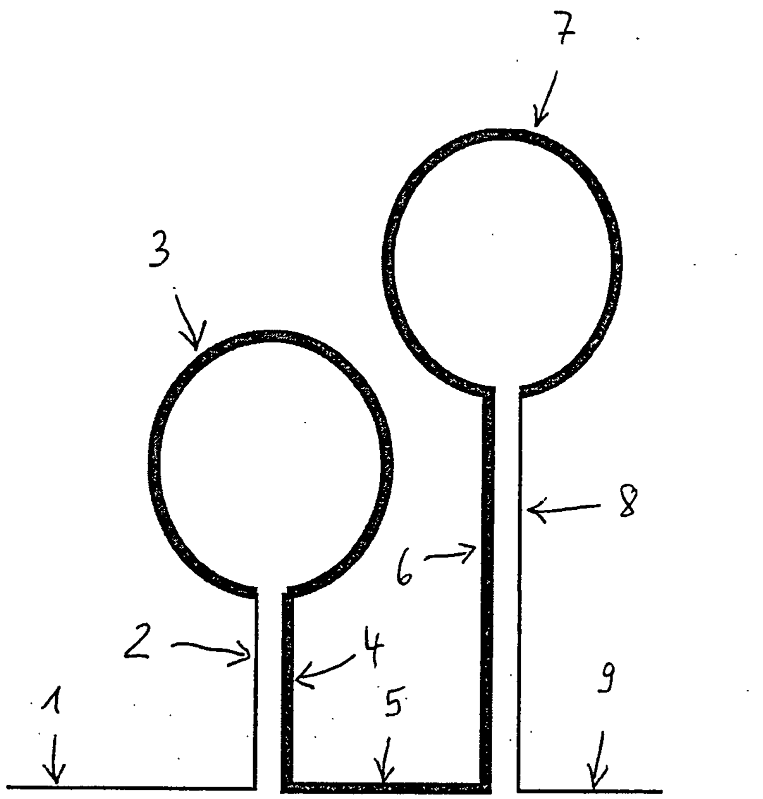


Figur 1

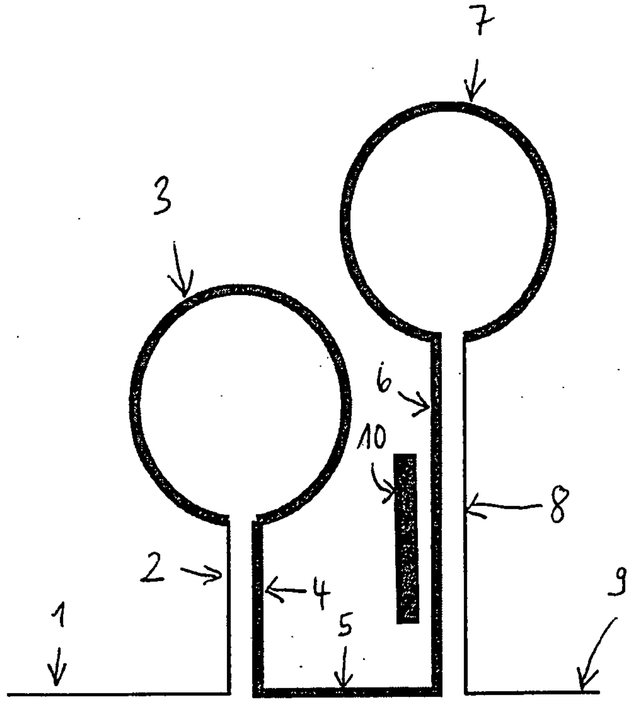


Figur 2

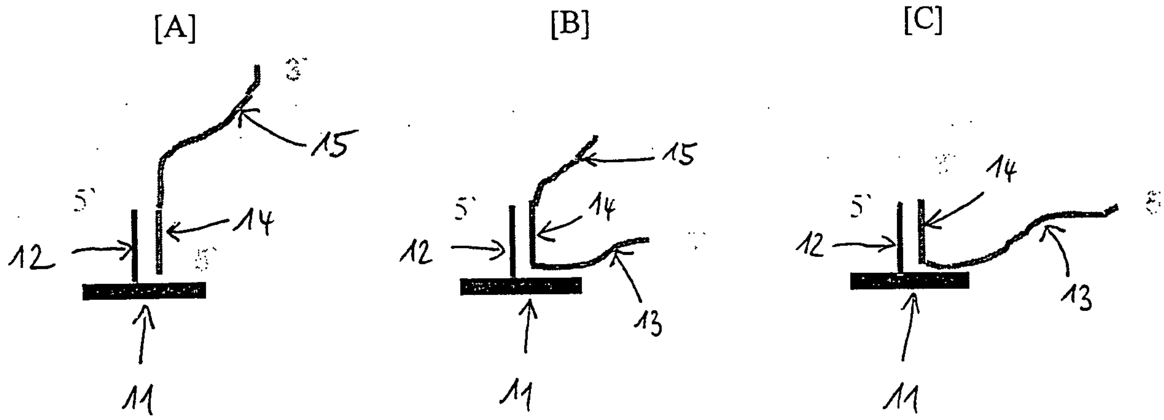


Figur 3

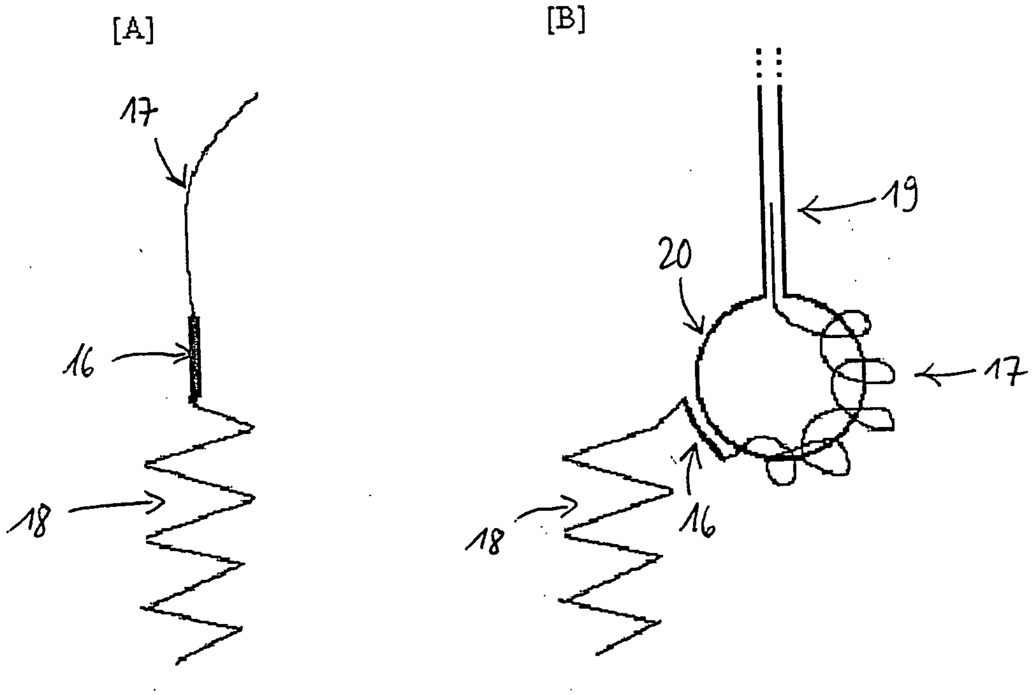


Figur 4

Europäisches Patentamt

**EUROPÄISCHER TEILRECHERCHENBERICHT**

der nach Regel 45 des Europäischen Patentübereinkommens für das weitere Verfahren als europäischer Recherchenbericht gilt

Nummer der Anmeldung
EP 01 11 8860

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| X | EBERHARDT NORMAN L: "A shell program for the design of PCR primers using genetics computer group (GCG) software (7.1) on VAX/VMS systems." BIOTECHNIQUES, Bd. 13, Nr. 6, 1992, Seiten 914-917, XP002204755 ISSN: 0736-6205 * Seite 915 - Seite 917; Abbildung 1; Tabelle 2 * --- | 1-10 | G06F19/00 C12Q1/68 |
| X | MITSUHASHI M ET AL: "OLIGONUCLEOTIDE PROBE DESIGN - A NEW APPROACH" NATURE, MACMILLAN JOURNALS LTD. LONDON, GB, Bd. 367, 24. Februar 1994 (1994-02-24), Seiten 759-761, XP002948495 ISSN: 0028-0836 * das ganze Dokument * --- -/-- | 1-10 | |
| | | | **RECHERCHIERTE SACHGEBIETE (Int.Cl.7)** |
| | | | G06F C12Q |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| DEN HAAG | 5. Juli 2002 | Gabriels, J |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

Europäisches Patentamt

**UNVOLLSTÄNDIGE RECHERCHE ERGÄNZUNGSBLATT C**

Nummer der Anmeldung
EP 01 11 8860

Nicht recherchierte Ansprüche:
11-13

Grund für die Beschränkung der Recherche (nicht patentfähige Erfindung(en)):

Artikel 52 (2)(c) EPÜ - Programm für Datenverarbeitungsanlagen

---

Weitere Beschränkung der Recherche

Nicht recherchierte Ansprüche:
14

Grund für die Beschränkung der Recherche:

Der geltende Patentanspruch 14 bezieht sich auf eine Vorrichtung, jeweils charakterisiert durch eine erstrebenswerte Eigenheit oder Eigenschaft, nämlich daß diese Vorrichtung ein Computerprogramm mit Programmcodemitteln umfaßt die zur Konfigurierung paralleler Nukleinsäureverfahren benutzt werden kann. Der Patentanspruch umfaßt daher alle Vorrichtungen etc., die diese Eigenheit oder Eigenschaft aufweisen, wohingegen die Patentanmeldung durch die Beschreibung im Sinne von Art. 83 EPÜ für keine solcher Vorrichtungen Stütze liefert. Im vorliegenden Fall fehlt den Patentanspruch der entsprechende Stütz bzw. der Patentanmeldung die nötige Offenbarung in einem solchen Maße, daß eine sinnvolle Recherche unmöglich erscheint. Desungeachtet fehlt den Patentansprüchen auch die in Art. 84 EPÜ geforderte Klarheit, nachdem in ihnen versucht die Vorrichtung über das jeweils erstrebte Ergebnis zu definieren. Auch dieser Mangel an Klarheit ist dergestalt, daß er eine sinnvolle Recherche unmöglich macht.

Europäisches Patentamt

**EUROPÄISCHER TEILRECHERCHENBERICHT**

Nummer der Anmeldung
EP 01 11 8860

| EINSCHLÄGIGE DOKUMENTE | | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| X | PEYRET NICOLAS ET AL: "Nearest-neighbor thermodynamics and NMR of DNA sequences with internal AcntdotA, CcntdotC, GcntdotG, and TcntdotT mismatches." BIOCHEMISTRY, Bd. 38, Nr. 12, 23. März 1999 (1999-03-23), Seiten 3468-3477, XP002204756 ISSN: 0006-2960 * Tabelle 1 * --- | 1-10 | |
| X | IGLOI GABOR L: "Variability in the stability of DNA-peptide nucleic acid (PNA) single-base mismatched duplexes: Real-time hybridization during affinity electrophoresis in PNA-containing gels." PROCEEDINGS OF THE NATIONAL ACADEMY OF SCIENCES OF THE UNITED STATES, Bd. 95, Nr. 15, 21. Juli 1998 (1998-07-21), Seiten 8562-8567, XP002204757 July 21, 1998 ISSN: 0027-8424 * Tabellen 1,2 * --- | 1-10 | RECHERCHIERTE SACHGEBIETE (Int.Cl.7) |
| X | SCHUETZ E ET AL: "SPREADSHEET SOFTWARE FOR THERMODYNAMIC MELTING POINT PREDICTION OF OLIGONUCLEOTIDE HYBRIDIZATION WITH AND WITHOUT MISMATCHES" BIOTECHNIQUES, EATON PUBLISHING, NATICK, US, Bd. 27, Nr. 6, Dezember 1999 (1999-12), Seiten 1218-1224, XP002948493 ISSN: 0736-6205 * das ganze Dokument * --- | 1-10 | |
| | -/-- | | |

EPO FORM 1503 03.82 (P04C12)

Europäisches Patentamt

# EUROPÄISCHER TEILRECHERCHENBERICHT

Nummer der Anmeldung
EP 01 11 8860

| EINSCHLÄGIGE DOKUMENTE | | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| X | HACIA J G ET AL: "DESIGN OF MODIFIED OLIGODEOXYRIBONUCLEOTIDE PROBES TO DETECT TELOMERE REPEAT SEQUENCES IN FISH ASSAYS" NUCLEIC ACIDS RESEARCH, OXFORD UNIVERSITY PRESS, SURREY, GB, Bd. 27, Nr. 20, 15. Oktober 1999 (1999-10-15), Seiten 4034-4039, XP000993421 ISSN: 0305-1048 * das ganze Dokument * | 1-10 | |
| X | BOMMARITO SALVATORE ET AL: "Thermodynamic parameters for DNA sequences with dangling ends." NUCLEIC ACIDS RESEARCH, Bd. 28, Nr. 9, 1. Mai 2000 (2000-05-01), Seiten 1929-1934, XP002204758 ISSN: 0305-1048 * Seite 1934 * | 1-10 | RECHERCHIERTE SACHGEBIETE (Int.Cl.7) |

EPO FORM 1503 03.82 (P04C12)